# EUROPEAN PATENT SPECIFICATION

(11) **EP 3 404 097 B1**
(45) Date of publication and mention of the grant of the patent: **02.09.2020**
(21) Application number: 18182700.7
(22) Date of filing: 01.07.2014
(51) Int. Cl.: C12N 9/14, C12N 9/88

(54) **EXPANDING THE HALOHYDRIN DEHALOGENASE ENZYME FAMILY: IDENTIFICATION OF NOVEL ENZYMES BY DATABASE MINING**
ERWEITERUNG DER HALOHYDRINDEHALOGENASEENZYMFAMILIE: IDENTIFIZIERUNG NEUARTIGER ENZYME DURCH DATENBANK-MINING
EXPANSION DE LA FAMILLE DES ENZYMES D'HALOHYDRINE DÉSHALOGÉNASE IDENTIFICATION DE NOUVELLES ENZYMES PAR EXPLORATION DE BASE DE DONNÉES

(43) Date of publication of application: 21.11.2018
(62) Divisional of application: 14175165.1
(73) Proprietor: Enzymicals AG, 17489 Greifswald (DE)
(72) Inventor: SCHALLMEY, Anett, 38124 Braunschweig (DE); SCHALLMEY, Marcus, 38124 Braunschweig (DE); WESSEL, Julia, 48493 Wettringen (DE); MENYES, Ulf, 17509 Neu Boltenhagen (DE); WARDENGA, Rainer, 17489 Greifswald (DE); BRUNDIEK, Henrike, 17493 Greifswald (DE); BORCHERT, Sonja, 1227 Carouge (CH)
(74) Representative: Altmann Stößel Dick Patentanwälte PartG mbB

(56) References cited:
- HYLCKAMA VLIEG VAN J E T ET AL: "Halohydrin dehalogenases are structurally and mechanistically related to short-chain dehydrogenases/reductases", JOURNAL OF BACTERIOLOGY, AMERICAN SOCIETY FOR MICROBIOLOGY, US, vol. 183, no. 17, 1 September 2001 (2001-09-01), pages 5058-5066, XP002305277, ISSN: 0021-9193, DOI: 10.1128/JB.183.17.5058-5066.2001
- JONG DE R M ET AL: "Structure and mechanism of a bacterial haloalcohol dehalogenase: A new variation of the short-chain dehydrogenase/reductase fold without an NAD(P)H binding site", EMBO JOURNAL, OXFORD UNIVERSITY PRESS, SURREY, GB, vol. 22, no. 19, 1 October 2003 (2003-10-01), pages 4933-4944, XP002305279, ISSN: 0261-4189, DOI: 10.1093/EMBOJ/CDG479
- ANETT SCHALLMEY ET AL: "Identifikation neuartiger Halohydrin-Dehalogenasen", BIOSPEKTRUM, vol. 19, no. 7, 19 November 2013 (2013-11-19), pages 816-817, XP055163271, ISSN: 0947-0867, DOI: 10.1007/s12268-013-0394-x
- DATABASE UniProt [Online] 6 March 2007 (2007-03-06), "SubName: Full=Putative oxidoreductase {ECO:0000313|EMBL:ABM93639.1};", XP055497264, retrieved from EBI accession no. UNIPROT:A2SDK0 Database accession no. A2SDK0
- DATABASE UniProt [Online] 16 April 2014 (2014-04-16), "SubName: Full=3-oxoacyl-[acyl-carrier-protein] reductase FabG {ECO:0000313|EMBL:EWS57318.1}; EC=1.1.1.100 {ECO:0000313|EMBL:EWS57318.1};", XP055497323, retrieved from EBI accession no. UNIPROT:W7W6Y0 Database accession no. W7W6Y0
- SCHALLMEY ET AL.,: "Expanding the halohydrin dehalgenase enzyme family: identification of novel enzymes by database mining", APPLIED AND ENVIRONMENTAL MICROBIOLOGY, vol. 80, no. 23, 19 September 2014 (2014-09-19), pages 7303-7315, XP009182061,

<110> Enzymicals AG
   Rheinisch-Westfälische Technische Hochschule (RWTH) Aachen
<120> Expanding the halohydrin dehalogenase enzyme family: Identification of novel enzymes by database mining
<130> EZY13965EP1
<160> 15
<170> PatentIn version 3.3
<210> 1
   <211> 227
   <212> PRT
   <213> marine metagenome
<400> 1
   Met Ser Lys Arg Leu Glu Gly Lys Arg Val Leu Val Thr Gln Ser Asn
   1               5                   10                  15
   Asn Tyr Met Gly Pro Ala Thr Val Glu Leu Phe Glu Lys Glu Gly Ala
               20                  25                  30
   Ile Val Thr Ala Asp Ser Ser Asp Leu Thr Glu Thr Glu Lys Cys Gln
           35                  40                  45
   Asp Ile Val Glu Ser Ala Gly Ile Ile Asp Ile Leu Ile Ala Asn Leu
       50                  55                  60
   Ala Ser Glu Asn Phe Ser Gly Ile Pro Thr Ser Asp Leu Thr Asp Glu
   65                  70                  75                  80
   Asp Trp Asn Thr Thr Phe Asp Val Met Val His Pro Leu His Lys Leu
                   85                  90                  95
   Ser Arg Ala Val Ile Pro Gln Met Val Glu Arg Gln Ala Gly Lys Ile
               100                 105                 110
   Ile Val Tyr Gly Ser Ala Ser Ala Leu Lys Gly Met Arg Thr Leu Ala
           115                 120                 125
   Ala Tyr Ser Ala Ala Arg Ala Ala Gln Val Gly Tyr Val Gln Ser Leu
       130                 135                 140
   Gly Val Glu Leu Ala Pro His Asn Ile Gln Val Asn Leu Ile Ala Gln
   145                 150                 155                 160
   Asn Tyr Val Glu Asn Pro Ile Tyr Tyr Pro Glu Ser Leu Arg Ser Gln
                   165                 170                 175
   Glu Lys Phe Gln Asn Ser Leu Lys Arg Gln Val Pro Leu Gly Arg Leu
               180                 185                 190
   Ala Arg Ala Glu Glu Asp Ala Gln Phe Ala Leu Phe Leu Ala Ser Asn
           195                 200                 205
   Glu Ser Asp Phe Phe Val Gly Gln Ala Ile Pro Phe Ser Gly Gly Trp
       210                 215                 220
   Val Gln Arg
   225
<210> 2
   <211> 224
   <212> **PRT**
   <213> marine metagenome
<400> 2
   Met Gly Arg Leu Asp Gly Lys Arg Val Leu Val Thr Gln Ala Asp Asp
   1               5                   10                  15
   Tyr Met Gly Pro Val Thr Leu Glu Val Phe Ala Glu Glu Gly Ala Glu
               20                  25                  30
   Val Ile Ala Asp Asn Ser Asp Leu Thr Asp Pro Ser Arg Ala Ala Ala
           35                  40                  45
   Leu Ile Glu Glu Thr Gly His Ile Asp Val Leu Val Ala Asn Leu Ala
       50                  55                  60
   Ala Pro Ala Asn Leu Gly Val Ala Ala Ala Asp Met Pro Asp Asp Ile
   65                  70                  75                  80
   Trp Gln Thr Met Phe Asp Val Met Val His Pro Leu His Arg Leu Thr
                   85                  90                  95
   Lys Ala Val Leu Pro Gln Met Val Glu Arg Gln Lys Gly Lys Ile Leu
               100                 105                 110
   Val Tyr Gly Ser Ala Thr Gly Val Lys Gly Met Ala Gly Ile Thr Ala
           115                 120                 125
   Tyr Ser Ala Ala Arg His Ala Gln Val Gly Tyr Val Arg Ser Thr Gly
       130                 135                 140
   Ala Glu Ile Ala Gly His Asn Ile Gln Met Asn Leu Ile Ala Gln Asn
   145                 150                 155                 160
   Phe Val Glu Asn Pro Val Tyr Phe Pro Pro Gln Phe Thr Glu Thr Pro
                   165                 170                 175
   Glu Phe Lys Glu Leu Leu Lys Gly Val Pro Ala Gly Arg Leu Ala Thr
               180                 185                 190
   Ala Arg Glu Asp Ala Leu Phe Ala Val Phe Leu Ala Ser Asp Glu Ser
           195                 200                 205
   Asp Phe Phe Val Gly Gln Ala Ile Pro Phe Ser Gly Gly Trp Ala Gln
       210                 215                 220
<210> 3
   <211> 226
   <212> PRT
   <213> marine metagenome
<400> 3
   Met Asp Lys Arg Leu Ala Gly Lys Arg Val Leu Ile Thr Gln Ala Glu
   1               5                   10                  15
   Asp Tyr Met Gly Pro Ala Ile Thr Glu Leu Phe Ala Glu His Gly Ala
               20                  25                  30
   Glu Ile Ile Ala Asp Thr Arg Asp Leu Thr Glu Asp Gly Ala Val Glu
           35                  40                  45
   Ser Leu Ile Lys Ser Ala Gly Glu Ile Asp Val Leu Ile Ala Asn Leu
       50                  55                  60
   Ala Ala Pro Ala His Leu Gly Leu Ser Val Thr Asp Thr Asp Asp Thr
   65                  70                  75                  80
   Thr Trp Glu Thr Ala Phe Asp Val Met Val His Pro Leu His Arg Ile
                   85                  90                  95
   Phe Arg Ser Val Leu Pro Ala Met Tyr Glu Arg Lys Arg Gly Lys Ile
               100                 105                 110
   Val Val Ile Gly Ser Ala Thr Gly Leu Lys Ala Leu Glu Gly Val Val
           115                 120                 125
   Ala Tyr Gly Ala Ala Arg His Ala Gln Val Gly Tyr Val Arg Ala Ala
       130                 135                 140
   Gly Ile Glu Ala Ala Arg His Asn Val Gln Ile Asn Leu Ile Ala Gln
   145                 150                 155                 160
   Asn Phe Val Glu Asn Pro Ala Tyr Phe Pro Pro Glu Phe Thr Ala Ser
   165 170 175
   Asn Glu Phe Lys Gln Leu Leu Ser Ala His Val Pro Leu Gly Arg Leu
   180 185 190
   Ala Thr Ala Arg Glu Asp Ala Leu Phe Ala Leu Phe Leu Ala Ser Asp
   195 200 205
   Glu Ser Asp Phe Phe Val Gly Gln Ala Ile Pro Phe Ser Gly Gly Trp
   210 215 220
   Ala Gln
   225
<210> 4
   <211> 226
   <212> PRT
   <213> marine metagenome
<400> 4
   Met Gly Asp Arg Leu Ala Gly Lys Arg Val Leu Val Thr Gln Ala Asp
   1 5 10 15
   Asn Tyr Met Gly Pro Ala Thr Ile Glu Leu Phe Thr Glu Glu Gly Ala
   20 25 30
   Glu Val Leu Ala Asp His Ser Asp Leu Thr Val Ala Gly Arg Cys Ser
   35 40 45
   Ala Leu Ile Lys Glu His Gly Arg Ile Asp Val Leu Val Ala Asn Leu
   50 55 60
   Ala Ser Pro Asn Phe Thr Gly Ile Pro Ala Thr Glu Leu Ala Asp Glu
   65 70 75 80
   Asp Trp His Cys Thr Phe Asn Met Met Val His Pro Leu His Gln Leu
   85 90 95
   Cys Gln Ala Val Val Pro Gln Met Ile Glu Arg Gln Ala Gly Lys Ile
   100 105 110
   Val Val Phe Gly Ser Ala Thr Ala Leu Lys Gly Met Pro Thr Val Thr
   115 120 125
   Ala Tyr Ser Ala Ala Arg Ala Ala Gln Ala Gly Tyr Val Arg Ser Leu
   130 135 140
   Gly Val Glu Ile Ala Lys His Asn Ile Gln Ala Asn Leu Ile Ala Gln
   145                 150                 155                 160
   Asn Tyr Val Glu Asn Pro Val Tyr Tyr Pro Pro Glu Leu Arg Glu Lys
                   165                 170                 175
   Glu Ser Phe Gln Lys Ser Leu Ala Arg Gln Val Pro Leu Gly Arg Leu
               180                 185                 190
   Ala Thr Ala Arg Glu Asp Ala Leu Phe Ala Leu Phe Leu Ala Ser Asp
           195                 200                 205
   Glu Ser Asp Phe Phe Val Gly Gln Ser Ile Ala Phe Ser Gly Gly Trp
       210                 215                 220
   Ala Gln
   225
<210> 5
   <211> 250
   <212> PRT
   <213> marine metagenome
<400> 5
   Met Ser Ser Asn Asp Leu Lys Gly Lys Arg Ile Leu Val Thr Gln Ala
   1               5                   10                  15
   Asp Thr Phe Met Gly Pro Thr Leu Cys Glu Val Phe Thr Glu Lys Gly
               20                  25                  30
   Ala Glu Val Ile Arg Asp Asn Gln Leu Leu Thr Glu Pro Thr Leu Pro
           35                  40                  45
   Ala Gln Leu Val Ala Ala Ala Gly Ser Ile Asp Val Leu Val Ile Asn
       50                  55                  60
   Leu Ala Ile Pro Ala Pro Phe Thr Lys Leu Glu Lys Val Asp Asp Asn
   65                  70                  75                  80
   Glu Trp Glu Ser Val Phe Ser Ala Val Val Asn Pro Met Pro Arg Leu
                   85                  90                  95
   Val Ser Ala Val Leu Pro Gln Met Ile Glu Arg Gln Ser Gly Lys Ile
               100                 105                 110
   Leu Val Met Gly Ser Ala Ser Ala Leu Arg Gly Met Lys Arg Ala Ser
           115                 120                 125
   Thr Tyr Ser Ala Ala Arg Gly Ala Gln Leu Ala Tyr Val Gln Ala Ala
       130                 135                 140
   Gly Val Glu Met Ala Pro His Asn Ile Gln Ile Asn Ala Ile Ala Gln
   145                 150                 155                 160
   Asn Phe Val Asp Asn Pro Thr Tyr Phe Pro Glu Glu Thr Lys Ala Asn
                   165                 170                 175
   Pro Ala Phe Gln Asp Arg Leu Lys Arg Glu Val Pro Leu Gly Arg Leu
               180                 185                 190
   Val Ser Met Arg Glu Asp Ala Gln Phe Ala Ala Tyr Leu Cys Ser Asp
           195                 200                 205
   Ala Ala Asp Cys Phe Val Gly Gln Val Phe Pro Val Ser Gly Gly Trp
       210                 215                 220
   Ala Gln Leu Lys Asn Glu Gln His Gly Met Ile Ile Ser Phe Ile Asp
   225                 230                 235                 240
   Ser Leu Ser Ala Lys Val Ile Arg Cys His
                   245                 250
<210> 6
   <211> 225
   <212> PRT
   <213> Limnohabitans sp. Rim28
<400> 6
   Met Ile Asp Leu Arg Gly Gln Arg Ile Leu Val Thr Gln Ala Gln Asp
   1               5                   10                  15
   Phe Met Gly Pro Ala Leu Cys Gln Glu Leu Arg Ala Cys Gly Ala Glu
               20                  25                  30
   Val Ile Ala Asp Asp Arg Val Leu Thr Ala Pro Gln Asp Ala Gln Ala
           35                  40                  45
   Met Ile Lys Glu Ala Gly Pro Leu Ala Ala Leu Val Ile Asn Leu Ala
       50                  55                  60
   Leu Pro Ala Pro Ser Thr Pro Val Thr Gln Ile Asp Glu Ala Glu Trp
   65                  70                  75                  80
   His Gln Val Phe Glu Val Met Val Asp Pro Leu Pro Arg Leu Val Arg
                   85                  90                  95
   Ala Val Val Pro Gly Met Lys Ala Arg Gly Gly Gly Lys Ile Val Val
   100 105 110
   Met Gly Ser Ala Ser Ala Leu Arg Gly Met Lys Arg Ala Ala Ser Tyr
   115 120 125
   Ser Ala Ala Arg Gly Ala Gln Leu Ala Tyr Val Gln Ala Ala Gly Val
   130 135 140
   Glu Leu Ala Pro Asp Asn Ile Gln Leu Asn Ala Val Ala Gln Asn Phe
   145 150 155 160
   Val Asp Asn Pro Thr Tyr Phe Pro Pro Glu Val Gln Ala Asn Pro Arg
   165 170 175
   Phe Gln Glu Arg Leu Lys Arg Glu Val Pro Leu Gly Arg Leu Val Lys
   180 185 190
   Ala Glu Glu Asp Ala Arg Phe Val Ala Tyr Leu Cys Ser Asp Ala Ala
   195 200 205
   Ser Cys Phe Val Gly Gln Val Phe Pro Met Ser Gly Gly Trp Ala Val
   210 215 220
   Arg
   225
<210> 7
   <211> 227
   <212> PRT
   <213> Curvibacter lanceolatus
<400> 7
   Met Ser Ser Gln Cys Leu Ala Gly Leu Arg Val Leu Ile Thr Gln Ala
   1 5 10 15
   Asn Glu Phe Met Gly Pro Thr Leu Cys Glu Val Phe Ala Glu Gln Gly
   20 25 30
   Ala Val Val Leu Ala Asp Asp Gly Pro Leu Thr Asp Pro Gln Ala Pro
   35 40 45
   Ala Arg Leu Val Ala Gly His Gly Pro Ile Asp Val Leu Val Ala Asn
   50 55 60
   Leu Ala Val Pro Ala Pro Ser Thr Pro Ala His Gln Val Ser Glu Gln
   65 70 75 80
   Glu Trp Arg Asp Thr Phe Ala Ala Leu Val Asp Pro Leu Pro Arg Leu
   85 90 95
   Cys Gln Ala Val Leu Pro Ala Met Met Ala Arg Arg Ser Gly Arg Ile
   100 105 110
   Leu Val Met Gly Ser Ala Ala Ala Leu Arg Gly Met Lys Arg Ser Ser
   115 120 125
   Thr Tyr Ser Ala Ala Arg Gly Ala Gln Leu Ala Tyr Val Gln Ala Leu
   130 135 140
   Gly Val Glu Val Ala Ser Tyr Asn Ile Gln Val Asn Ala Ile Ala Gln
   145 150 155 160
   Asn Leu Val Asp Asn Pro Thr Tyr Phe Pro Pro Glu Val Gln Ala Asp
   165 170 175
   Pro Arg Phe Gln Glu Arg Leu Arg Arg Glu Val Pro Leu Gly Arg Leu
   180 185 190
   Val Ser Ala Arg Glu Asp Ala Glu Phe Ala Ala Tyr Leu Cys Ser Ala
   195 200 205
   Pro Ala Ala Cys Phe Val Gly Gln Val Phe Pro Val Cys Gly Gly Trp
   210 215 220
   Val Gly Arg
   225
<210> 8
   <211> 225
   <212> PRT
   <213> marine metagenome
<400> 8
   Met Arg Leu Glu Asn Lys Arg Val Met Val Thr Gln Ser Asp Asp Tyr
   1 5 10 15
   Met Gly Pro Ala Ile Thr Ser Leu Phe Ser Thr Glu Gly Ala Gln Val
   20 25 30
   Thr Thr Arg Glu Lys Pro Val Pro Thr Gly Lys Ala Phe Ser Thr Trp
   35 40 45
   Val Arg Glu Met Pro Val Tyr Asp Val Val Val Ala Asn Leu Ala His
   50 55 60
   Asp Pro Cys Ser Ser Ala Val Asp Asn Ile Asp Asn Glu Asp Trp Gln
   65 70 75 80
   Ala Leu Phe Glu Thr Leu Val His Pro Leu Met Tyr Leu Val Arg His
   85 90 95
   Phe Ala Pro Lys Met Ala Glu Arg Gly Tyr Gly Lys Ile Ile Ala Ile
   100 105 110
   Thr Ser Ala Ala Pro Leu Arg Gly Ile Pro Gly Ser Thr Ala Tyr Cys
   115 120 125
   Ala Ala Arg Gly Ala Gln Asn Ala Phe Ile Arg Ala Thr Gly Leu Glu
   130 135 140
   Phe Ala Ala Arg Gly Val Asn Ile Asn Ala Val Ala Gln Asn Tyr Val
   145 150 155 160
   Ser Asn Pro Ala Tyr Phe Pro Asp Asp Leu Val Ala Ser Glu Arg Phe
   165 170 175
   Gln Lys His Leu Ala Arg Asn Val Pro Ile Gly Arg Val Ala Lys Asp
   180 185 190
   Thr Glu Ser Ala Glu Leu Ala Leu Phe Leu Ala Ser Asn Ala Ser Asp
   195 200 205
   Phe Ile Val Gly Gln Val Val Pro Phe Ser Gly Gly Trp Ala Thr Asn
   210 215 220
   Thr
   225
<210> 9
   <211> 226
   <212> PRT
   <213> marine metagenome
<400> 9
   Met Thr Arg Leu Glu His Lys Lys Val Leu Ile Thr Gln Ser Asp Asp
   1 5 10 15
   Tyr Met Gly Pro Ala Ile Ala Asp Leu Phe Ala Ala Glu Ala Ala Arg
   20 25 30
   Val Thr Ala Arg Pro Gly Leu Val Pro Phe Gly Thr Gln Phe Ala Arg
   35 40 45
   Tyr Val Gln Gly Leu Pro Asp Phe Asp Val Val Ile Ala Asn Leu Ala
       50                  55                  60
   His Asp Pro Cys Asn Gly Pro Ile Glu Thr Leu Ala Asp Glu Ser Trp
   65                  70                  75                  80
   Glu Lys Leu Phe Asp Thr Met Val His Pro Leu Met Ala Leu Val Arg
                   85                  90                  95
   His Phe Ala Pro Arg Met Ala Asp Gln Gly His Gly Lys Ile Ile Ala
               100                 105                 110
   Ile Thr Ser Ala Ala Pro Leu Lys Gly Leu Pro Gly Ser Ala Ala Tyr
           115                 120                 125
   Cys Ala Ala Arg Gly Ala Gln Asn Ala Phe Ile Lys Ala Val Gly Leu
       130                 135                 140
   Glu Phe Ala Ala Lys Asn Val Gln Ile Asn Ala Ile Ala Gln Asn Tyr
   145                 150                 155                 160
   Val Ser Asn Pro Val Tyr Tyr Pro Asp Glu Leu Val Gln Ser Glu Arg
                   165                 170                 175
   Phe Gln Lys His Leu Ala Arg Asn Val Pro Ile Arg Arg Val Ala Arg
               180                 185                 190
   Pro Glu Glu Gln Ala Glu Phe Ala Leu Phe Leu Ala Ser Asn Asn Ser
           195                 200                 205
   Asp Phe Phe Val Gly Gln Ile Phe Pro Phe Ser Gly Gly Trp Ala Ile
       210                 215                 220
   Asn Ala
   225
<210> 10
   <211> 222
   <212> PRT
   <213> gamma proteobacterium IMCC3088
<400> 10
   Met Ser Gln Phe Ser Gly Lys Ser Val Trp Val Thr Ser Ala Asp Arg
   1               5                   10                  15
   Tyr Met Gly Pro Ser Ile Ala Asp Glu Phe Glu Arg Leu Gly Ala Ile
               20                  25                  30
   Val Thr Arg Asp Met His Val Leu Tyr Asp Asp His Tyr Leu Arg Glu
   35 40 45
   Thr Leu Ala Glu Ile Pro Asp Ile Val Ile Ala Asn Leu Ala Glu Pro
   50 55 60
   Pro Arg Lys Asp Ala Leu Glu Ala Ile Gln Asp Asp Asp Trp Asn Leu
   65 70 75 80
   Leu Phe Asp His Leu Val His Pro Leu Met Arg Ile Val Arg His Val
   85 90 95
   Ser Gly Pro Met Lys Ala Arg Gly His Gly Lys Ile Val Ala Ile Thr
   100 105 110
   Ser Ala Ala Pro Leu Arg Gly Ile Pro Phe Ala Ser Gly Tyr Cys Ala
   115 120 125
   Ala Arg Gly Ala Gln Asn Ala Phe Ile Lys Gly Ala Gly Leu Glu Leu
   130 135 140
   Ala Lys Phe Gly Val Gln Ala Asn Ala Ile Gly Gln Asn Tyr Ile Glu
   145 150 155 160
   Asn Asp Thr Tyr Tyr Pro Pro Glu Leu Met Gln Asp Pro Arg Phe Ile
   165 170 175
   Ser Asn Leu Ser Ser Gln Val Pro Thr Lys Lys Val Gly Arg Gly Leu
   180 185 190
   Glu Thr Ala Lys Leu Ala Ala Tyr Leu Ala Asp Pro Asp Val Glu His
   195 200 205
   Val Val Gly Gln Ile Ile Pro Leu Ala Gly Gly Trp Thr Thr
   210 215 220
<210> 11
   <211> 226
   <212> PRT
   <213> marine gamma proteobacterium HTCC2143
<400> 11
   Met Arg Tyr Ser Asn Lys Arg Val Leu Val Thr Thr Ser Asn Arg Tyr
   1 5 10 15
   Met Gly Pro Asp Ile Ala Thr Arg Phe Glu Glu Glu Gly Ala Glu Val
   20 25 30
   Ile Arg Ser Glu Asn Arg Leu Leu Asp Gln Gln Ser Val Asp Ala Leu
   35 40 45
   Ile Glu Ser Val Gly Glu Val Asp Val Leu Val Ala Asn Phe Ala Glu
   50 55 60
   Pro Pro Arg Thr Ala Pro Val Glu Lys Ile Ser Asp Glu Asp Trp Phe
   65 70 75 80
   Ile Leu Phe Asp Thr Leu Val His Pro Leu Met Arg Thr Val Arg Gly
   85 90 95
   Phe Ala Gly Val Met Lys Glu Arg Arg Arg Gly Lys Ile Ile Ala Val
   100 105 110
   Thr Ser Ala Ala Pro Leu Arg Gly Ile Pro Asn Ala Ala Gly Tyr Cys
   115 120 125
   Ala Ala Arg Gly Ala Gln Asn Ala Phe Val Arg Ala Val Gly Leu Glu
   130 135 140
   Leu Ala Lys Ser Asn Val Gln Phe Asn Ala Ile Ala Gln Asn Tyr Ile
   145 150 155 160
   Ser Asn Asp Thr Tyr Tyr Pro Asp Asp Phe Val Ala Asn Glu Lys Phe
   165 170 175
   Lys Ala His Leu Lys Asp Phe Val Pro Thr Lys Lys Val Ala Asp Gly
   180 185 190
   Ala Glu Thr Ala Glu Leu Ala Leu Tyr Leu Ala Ser Asp Leu Cys Thr
   195 200 205
   His Leu Val Gly Gln Val Ile Pro Phe Ser Gly Gly Trp Ala Thr Thr
   210 215 220
   Thr Gly
   225
<210> 12
   <211> 219
   <212> PRT
   <213> marine metagenome
<400> 12
   Met Ser Arg Arg Thr Ala Leu Val Thr Cys Ile Asp Ser Tyr Met Gly
   1 5 10 15
   Pro Ala Ile Ser Glu Lys Phe Glu Glu Ile Gly Ile Asn Val Ile Ala
               20                  25                  30
   Gly Pro Ser Asp Leu His Ser Glu Ala Gln Ile Asn Lys Leu Val Ala
           35                  40                  45
   Asp Ala Gly His Ile Asp Ile Leu Val Ala Asn Leu Ala Glu Pro Pro
       50                  55                  60
   Met Thr Gly Pro Val Gln Asn Ile Asp Ser Asp Asn Trp Asn Leu Leu
   65                  70                  75                  80
   Phe Asp Ser Leu Val His Pro Leu Met Tyr Leu Val Arg Ala Val Thr
                   85                  90                  95
   Pro Gln Met Leu Glu Arg Lys Ser Gly Lys Ile Ile Ala Ile Thr Ser
               100                 105                 110
   Ala Ala Pro Leu Arg Gly Ile Pro Asn Asn Ala Ala Tyr Cys Ala Ala
           115                 120                 125
   Arg Gly Ala Gln Asn Gly Phe Ile Lys Ala Ala Gly Leu Glu Leu Ala
       130                 135                 140
   Arg Ser Asn Ile Gln Val Asn Ala Ile Ala Gln Asn Tyr Ile Asn Asn
   145                 150                 155                 160
   Asn Thr Tyr Tyr Pro Asp Asp Ile Leu Asp Asn Gln Lys Phe Leu Asn
                   165                 170                 175
   His Val Lys Arg Asn Val Pro Ser Asn Lys Ile Gly Ala Ala Ser Glu
               180                 185                 190
   Thr Ala Gln Leu Ala Ala Tyr Leu Ser Arg Glu Asp Cys Arg His Met
           195                 200                 205
   Val Gly Gln Val Ile Pro Leu Ala Gly Gly Trp
       210                 215
<210> 13
   <211> 219
   <212> PRT
   <213> marine metagenome
<400> 13
   Met Ala Val Leu Val Thr His Gly Asp Arg Tyr Met Gly Pro Ala Ile
   1               5                   10                  15
   Ala Asp Arg Phe Glu Ala Ala Gly Ala Leu Val Thr Arg Asp Val Thr
               20                  25                  30
   Gln Pro Thr Ser Ala Glu Ala Ala Glu Ala Leu Val Glu Gly Gln Glu
           35                  40                  45
   Ala Leu Asp Ile Leu Ile Ala Asn Leu Ala Tyr Pro Pro Thr Pro Ala
       50                  55                  60
   Leu Val Asp Ala Ile Asp Ser Asp Gly Trp His Ala Leu Phe Asp His
   65                  70                  75                  80
   Leu Val His Pro Leu Met Gly Leu Val Arg Gly Ala Ala Lys Arg Met
                   85                  90                  95
   Lys Ala Gln Gly His Gly Arg Ile Ile Ala Ile Thr Ser Ala Ala Pro
               100                 105                 110
   Leu Arg Gly Ile Pro Lys Asn Gly Ala Tyr Cys Ala Ala Arg Gly Ala
           115                 120                 125
   Gln Asn Ala Phe Leu Arg Ala Ala Gly Leu Glu Leu Ala Arg Asp Gly
       130                 135                 140
   Ile Ala Val Ala Ala Ile Ala Gln Asn Tyr Ile Glu Asn Asp Thr Tyr
   145                 150                 155                 160
   Tyr Pro Pro Glu Leu Leu Asp Asp Glu Arg Phe Leu Ala Arg Met Arg
                   165                 170                 175
   Glu Val Val Pro Ala Gln Gln Ile Gly Ala Pro Ser Ala Thr Ala Asn
               180                 185                 190
   Leu Ala Tyr Phe Leu Ala Thr Glu Ala Glu Phe Met Pro Gly Gln Ile
           195                 200                 205
   Phe Pro Leu Ser Gly Gly Trp Thr Thr Thr Leu
       210                 215
<210> 14
   <211> 231
   <212> PRT
   <213> uncultured bacterium
<400> 14
   Met Thr Glu Gln Pro Gln Lys Asn Gly Tyr Gly Leu Ser Gly Lys Arg
   1               5                   10                  15
   Val Val Ile Thr Gln Ala Ala Gly Phe Met Gly Pro Ser Leu Val Glu
   20 25 30
   Ala Phe Ser Arg Glu Gly Ala Glu Val Ile Pro Asp His Arg Asp Leu
   35 40 45
   Thr His Asp Lys Ala Ala Asp Asn Leu Val Ser Glu Phe Lys Glu Ile
   50 55 60
   Asp Ile Leu Leu Ile Asn Leu Ala Ser Gln Arg Gln Arg Ile Glu Ala
   65 70 75 80
   Thr Glu Ile Ser Asp Gln Gln Phe Leu Gln Pro Phe Glu Glu Met Val
   85 90 95
   Tyr Pro Leu Phe Arg Leu Gly Arg Ser Val Leu Pro Gln Met Ile Ala
   100 105 110
   Arg Arg Arg Gly Lys Ile Ile Val Ile Gly Ser Ala Ala Pro Leu Arg
   115 120 125
   Pro Phe Ala Asn Ala Thr Gly Tyr Ala Thr Ala Arg Gly Ala Gln Leu
   130 135 140
   Ala Trp Val Lys Ala Val Gly Ala Glu Val Ala Gln His Asn Val Gln
   145 150 155 160
   Val Asn Gly Ile Ala Gln Ile Phe Val Glu Asn Gln Glu Tyr Phe Pro
   165 170 175
   Pro Ala Tyr Leu Gln Thr Asp Glu Phe Lys Gln Arg Ile Ala Gln Val
   180 185 190
   Pro Ala Gly Arg Leu Gly Ser Ala Ala Glu His Ala Ala Leu Ser Leu
   195 200 205
   Phe Leu Ala Ser Asp Gln Cys Asn Phe Ile Ser Gly Gln Val Val Pro
   210 215 220
   Phe Ala Gly Gly Trp Thr Thr
   225 230
<210> 15
   <211> 227
   <212> PRT
   <213> uncultured marine bacterium Ant4E12
<400> 15
   Met Ser Gln Arg Leu Ala Asn Lys Arg Val Val Val Thr Asn Ala Asn
   1               5                   10                  15
   Asp Phe Met Gly Ala Pro Thr Ile Glu Val Phe Glu Glu Glu Gly Ala
               20                  25                  30
   Glu Val Ile Ala Asp Tyr Ser Asn Leu Met Asp Ser Asp Ala Ala Ser
           35                  40                  45
   Thr Leu Ile Glu Met Val Gly Gln Val Asp Val Leu Val Val Asn Leu
       50                  55                  60
   Ala Ala Pro Ser Phe Gly Gly Ile Pro Thr His Glu Leu Asn Asp Glu
   65                  70                  75                  80
   Asn Phe Ala Glu Cys Phe Asp Val Met Val Tyr Pro Leu His Arg Leu
                   85                  90                  95
   Thr Arg Ala Val Leu Pro Gln Met Ile Glu Arg Lys Ala Gly Lys Ile
               100                 105                 110
   Val Val Tyr Gly Ser Ala Thr Ala Leu Lys Gly Met Lys Thr Leu Ala
           115                 120                 125
   Ala Tyr Ser Ala Ala Arg Gly Ala Gln Val Ser Tyr Val Gln Ser Val
       130                 135                 140
   Gly Ile Glu Val Ala Pro Gln Asn Ile Gln Met Asn Leu Ile Ala Gln
   145                 150                 155                 160
   Asn Tyr Val Glu Asn Pro Asp Tyr Tyr Pro His Glu Leu Thr Ser Gln
                   165                 170                 175
   Glu Ser Phe Lys Ser Ser Leu Lys Arg Gln Val Pro Leu Gly Arg Leu
               180                 185                 190
   Ala Thr Ser Arg Glu Asp Ala Gln Leu Ala Ala Phe Leu Ala Ser Asn
           195                 200                 205
   Glu Ser Asp Phe Phe Val Gly Gln Ala Ile Pro Phe Ala Gly Gly Trp
       210                 215                 220
   Val Gln Arg
   225

## Description

Halohydrin dehalogenases (also called haloalcohol dehalogenases, haloalcohol/halohydrin epoxidases, or hydrogen-halide lyases; EC 4.5.1.-) (HHDHs) are biotechnologically relevant enzymes that catalyze the reversible dehalogenation of β-haloalcohols under epoxide formation (1-3). Besides being useful for the production of enantiopure haloalcohols (4-6) and epoxides (5, 7-9), these enzymes can also be applied in the formation of novel carbon-carbon, carbon-nitrogen, or carbon-oxygen bonds. This is due to their promiscuous epoxide ring-opening activity accepting also other nucleophiles (e.g. cyanide, azide, or nitrite) beside halides which leads to a diverse range of products (10). Examples of relevant HHDH applications are the production of optically pure C3 or C4 fine chemical precursors (5, 8, 11, 12), including the multi-ton scale production of enantiopure (R)-4-cyano-3-hydroxybutyrate esters for statin drugs (13) or the production of chiral tertiary alcohols (14-17) for which conventional organic synthesis is rather challenging.

Despite their designated potential as biocatalysts, only few HHDHs have been made available. Although many bacterial species have been reported to possess HHDH activity, only six HHDH genes have been cloned and expressed recombinantly, namely *hheA* from *Corynebacterium* sp. strain N-1074 (18), *hheA2* from *Arthrobacter* sp. strain AD2 (19), *hheB* from *Corynebacterium* sp. strain N-1074 (18), *hheB2* from *Mycobacterium* sp. strain GP1 (19), and two identical *hheC* sequences from *Agrobacterium radiobacter* AD1 (19) and *Rhizobium* sp. strain NHG3 (20).

All of the cloned HHDHs belong to the short-chain dehydrogenase/reductase (SDR) superfamily and exhibit several major features of this diverse enzyme class (19, 21, 22). For example, all known HHDHs make use of a catalytic triad of Ser-Tyr-Arg, share the commonly found homomultimeric quarternary assembly and both crystallized HHDHs, namely HheA2 (23) and HheC (24), possess a tertiary structure similar to other Rossmann-fold proteins. In contrast of a cofactor-binding site present in SDR enzymes, a spacious anion binding pocket is present in the structures of HheA2 and HheC (23, 24). In consequence, all known HHDH sequences form only a minute but well-defined fraction within the SDR superfamily.

Although these few known HHDHs have already given rise to many interesting applications, it is of great interest to increase the number of functionally diverse HHDH enzymes. Clearly, novel sequences would be a valuable addition to the functional diversity of the HHDH enzyme toolbox. Public sequence databases hold an immense treasure of sequences with only limited or without any information about the function of these sequences or their related biomolecules. Schallmey et al. discloses the identification of new Halohydrin dehalogenases using database mining of publicly available sequence databases using HHDH-specific sequence information (A).

The object of the present invention was the use of newly as HHDHs identified enzymes among already deposited sequences.

The problem was solved by the use of a biomolecule having the protein sequence or coding DNA sequence of HheD3 as halohydrin dehalogenase according to claim 1.
a) A method for the identification of enzymes having potential halohydrin dehalogenase activity from sequences disclosed in a biomolecule database - which is not covered by the present invention- comprises the following steps:providing at least one biomolecule database,
b) searching the biomolecule database to identify at least one target biomolecule sequence, which comprises, on amino acid level, both of the following amino acid sequence motifs (1) and (2) anywhere within the target biomolecule sequence
   (1) amino acid Threonine (T) being followed by four variable amino acids (x(4)), followed by amino acid Phenylalanine (F) or Tyrosine (Y) followed by one variable amino acid (x), followed by amino acid Glycine (G):

      "T-x(4)-[FY]-x-G"

      and
   (2) amino acid Serine (S) followed by 12 variable amino acids (x(12)), followed by amino acid Tyrosine (Y), followed by three variable amino acids (x(3)), followed by amino acid Arginine (R):

      "S-x(12)-Y-x(3)-R",

      wherein in (1) and (2) variable amino acids (x) can independently be the same or different and are selected from natural and synthetic amino acids.

The sequences are always indicated from N- to C-terminus for proteins or in case of DNA from 5' to 3'-terminus. The indication of amino acids within square brackets [] means that the amino acids within the brackets are interchangeable. For example, "[FY]" means that F can be replaced by Y. The wordings "motif and "sequence pattern" are used synonymously. The one-letter-abbreviation as well as the three-letter-abbreviation of amino acids complies with the standard.

A biomolecule database is a source of biomolecules itself or a collection of sequence data of biomolecules, in particular polynucleotide or polypeptide sequence data. In the context of the present invention a biomolecule is preferably a polynucleotide molecule carrying genetic information, in particular a DNA molecule. In a further preferred embodiment of the present invention a biomolecule is a polypeptide, in particular a protein, comprising a number of amino acids (protein ≥100 amino acids). Thus, a biomolecule database may either be a physical source of biomolecules, in particular may be a gene bank, in particular a cDNA- or genomic library or is a collection of information about said biomolecules, in particular a collection of sequence data, in particular amino acid sequences or polynucleotide sequences, in particular DNA sequences.

An additional motif (3) can be present anywhere in the target biomolecule sequence, wherein amino acid Glycine (G) is followed by six variable amino acids (x(6)), followed by two Glycines (G)

"G-x(6)-G-G"

Especially, the method for the identification of enzymes having potential halohydrin dehalogenase activity from sequences disclosed in a biomolecule database comprises an additional step
c) providing the at least one biomolecule having the target biomolecule sequence identified in step b) and being or encoding at least partially a protein with the activity of a halohydrin dehalogenase and, optionally, an additional step
d) proving/verifying halohydrin dehalogenase activity of the at least one biomolecule having the target biomolecule sequence by conversion of vicinal halohydrins to epoxides or by ring opening of epoxides with nucleophiles.

"Proving/verifying halohydrin dehalogenase activity" means that the target biomolecule at least partially converts 1,3-dihalo-2-propanol (halo = chloro, bromo, iodo, preferably 1,3-dichloro-2-propanol) as standard vicinal halohydrin into the corresponding epihalohydrin (halo = chloro, bromo, iodo, preferably epichlorohydrin) and/or converts at least partially epihalohydrin (halo = chloro, bromo, iodo, preferably epichlorohydrin) or glycidol as standard epoxides with halide ions (Cl⁻, I⁻, Br⁻, preferably Cl⁻) or azide ion N₃⁻ as standard nucleophiles.

The term "a biomolecule being or coding at least partially a protein" means preferably that the biomolecule may either be a protein with the activity of a halohydrin dehalogenase or, in case the biomolecule is a nucleotide sequence, at least a part of said nucleotide sequence codes said protein, preferably a full-length protein. Accordingly, in case the provided biomolecule is a nucleotide sequence molecule, at least a part of said nucleotide sequence molecule codes a protein with the activity of a halohydrin dehalogenase, whereby possibly a further part of said nucleotide molecule has some other function, for instance regulatory or replicative function. Thus, the term "providing a biomolecule having the target biomolecule sequence identified in step b) and being or coding at least partially a protein with the activity of a halohydrin dehalogenase" is preferably equivalent to the term "providing a biomolecule having the target biomolecule sequence identified in step b) which may be a protein with the activity of a halohydrin dehalogenase or which may be a nucleotide sequence molecule which includes a sequence coding said protein as long as the protein has the activity of a halohdrin dehalogenase".

Possible with respect to the identification is that motif (1) and motif (2) are spaced from each other by 1 to 250 variable amino acids (x(1,250))

"T-x(4)-[FY]-x-G-x(1,250)-S-x(12)-Y-x(3)-R",

more preferably by 50 to 200 variable amino acids (x(50,200)), most preferably by 93 to 131 variable amino acids (x(93,131)), wherein variable amino acids (x) can be the same or different and are selected from natural and synthetic amino acids.

As mentioned previously, motifs (1), (2) follow each other from N- to C-terminus or in case of DNA from 5' to 3'-terminus.

If (3) is present, it is preferably spaced from motif (2) by 1 to 250 variable amino acids (x(1,250))

S-x(12)-Y-x(3)-R-x(1,250)-G-x(6)-G-G"

more preferably by 50 to 200 variable amino acids (x(50,200)), most preferably by 76 to 84 variable amino acids (x(76,84)), wherein variable amino acids (x) can be the same or different and are selected from natural and synthetic amino acids.

Possible with respect to the identification is that motifs (1), (2), (3) follow each other from N- to C-terminus or in case of DNA from 5' to 3'-terminus:

"T-x(4)-[FY]-x-G-x(1,250)-S-x(12)-Y-x(3)-R-x(1,250)-G-x(6)-G-G",

wherein preferred variants of the spacing amino acids x(1,250) between motifs (1) and (2) and between (2) and (3) are both as defined above. Motif (3) is of special use to ensure that the enzyme is functional, i.e. that the sequence is complete at the C-terminus. Without being bound to this theory: motif (3) might be essential for the formation of oligomers.

In addition, the presence of motif (1) also ensures that the enzyme is functional, i.e. that the sequence is complete at the N- terminus. It is possible that motif (3) is followed by 0-40 amino acids, preferred by 1-30 amino acids. Additionally, 0-40 amino acids, more preferred 1-30 amino acids may precede motif (1).

The biomolecules are proteins or DNA molecules and the biomolecule sequences are amino acid sequences or DNA sequences. Proteins and thus amino acid sequences are preferred.

Possible with respect to the identification is that step a) consists of two steps a1) and a2)
a1) providing the at least one biomolecule database (e.g. GenBank, UniProt, PFAM, Prosite)
a2) providing at least one query biomolecule sequence of at least one halohydrin dehalogenase wherein steps a1) and a2) are interchangeable in order and
step b) consists of two successive steps b1) and b2),
b1) searching the biomolecule database with the at least one query biomolecule sequence to identify a group of pre-target biomolecule sequences,
b2) selecting from the group of pre-target biomolecule sequences at least one target biomolecule sequence, which comprises, on amino acid level, both of the motifs (1) and (2) anywhere within the at least one target biomolecule sequence and optionally comprises motif (3) anywhere within the at least one target biomolecule sequence.

Preferably, all three motifs (1), (2) and (3) have to be present.

The identification method may be in its steps a) to b) applicable to a biomolecule database being a collection of sequence data ofpolynucleotide sequences, in particular DNA sequences or amino acid sequences, both of which are searched in a step b1) with sequence alignment tools. This especially means BLAST (**B**asic **L**ocal **A**lignment **S**earch **T**ool, BLASTp (p=protein) and/or BLASTn (n=nucleotide)). Other suitable programs/tools include pairwise sequence alignment and multiple sequence alignment programs such as MAFFT, GAP, BESTFIT, MUSCLE, CLUSTALW (and successors), T-COFFEE (and flavors), PRANK and FASTA.

In case of the query biomolecule sequence being a DNA sequence, said sequence is preferably an open reading frame (ORF) of the query biomolecule coding sequence or a characteristic part thereof. In case of the query biomolecule sequence being a protein sequence, said sequence is preferably the ORF-amino acid sequence itself or a characteristic part thereof.

It has to be noted that the above-defined special variant of the identification method is related to the use of a query biomolecule sequence as starting point for the search within a biomolecule database. However, alternative variants of the identification method are possible where a biomolecule bank is searched merely based on the motifs (1) and (2) and if needed (3). Such motif-only searches are available, for example, via ScanProsite and MAST (included in the MEME suite).

The group of pre-target biomolecules mentioned in steps b1), b2) might also consist of only one biomolecule, i.e. the target biomolecule. If there is only one pre-target biomolecule, no selection from a group is necessary.

Below, naming for genes and enzymes of the HHDH enzyme family (Hhe) is done on the basis of their clustering to any of the phylogenetic subtypes A through G (HheA-HheG) and, if necessary, additional phylogenetic subtypes. Then, numbering of enzymes within each subtype was done consecutive according to the time of submission to any of the public databases.

Since searching of biomolecule databases automatically also results in finding of already as HHDH indicated sequences, sequences of HheA, HheA2, HheB, HheB2, HheC (group 1a) are excluded as pre-target biomolecule sequences or target biomolecule sequences. HheA, HheA2, HheB, HheB2, HheC are known HHDHs and their activity as HHDH is proven. Additionally, HheA4 has a sequence identity on amino acid level of 99% with HheA, even if there is no proof of HHDH activity or naming as HHDH in literature for HheA4. A special variant thus excludes HheA, HheA2, HheA4, HheB, HheB2, HheC (group 2a) as pre-target biomolecule sequences or target biomolecule sequences. HheA5 is merely named as HHDH in biomolecule databases without reliable proof of real HHDH activity. HheA3 belongs to an automatically annotated cluster of HHDH-like sequences (CDD, conserved domain database) without reliable proof of real HHDH activity. A more special variant thus excludes HheA, HheA2, HheA3, HheA4, HheA5, HheB, HheB2, HheC (group 3a) as pre-target biomolecule sequences or target biomolecule sequences. The most special variant additionally excludes HheGx and HheBx, i.e. overall excludes HheA, HheA2, HheA3, HheA4, HheA5, HheB, HheB2, HheBx, HheC, HheGx (group 4a) as pre-target biomolecule sequences or target biomolecule sequences, because HheGx has an M instead of a T in motif (1) and the annotated sequence ABC25271 (GenBank), which corresponds partially to the HheBx sequence, is void of motif (1) (which leads to an enzyme without HHDH activity) but the latter is merely named as HHDH in biomolecule databases without reliable proof of real HHDH activity.

One possible variant excludes as the at least one query biomolecule sequence and/or as pre-target biomolecule sequence and/ or target biomolecule sequence sequences having less or coding for less than or equal to 186 amino acids. In other words, the at least one query biomolecule sequence and/or pre-target biomolecule sequence and/ or target biomolecule sequence has to have more than 186 amino acids or has to code for more than 186 amino acids.

Another possible variant excludes as the at least one query biomolecule sequence and/or as pre-target biomolecule sequence and/ or target biomolecule sequence sequences having less or coding for less than or equal to 201 amino acids. In other words, the at least one query biomolecule sequence and/or pre-target biomolecule sequence and/ or target biomolecule sequence has to have more than 201 amino acids or has to code for more than 201 amino acids.

In one possible aspect of the identification method, where at least one query biomolecule sequence of at least one halohydrin dehalogenase is used, i.e. steps a1) and a2) are used, the at least one query biomolecule sequence of at least one halohydrin dehalogenase is selected from protein sequences or coding DNA sequences of HheA, HheA2, HheA3, HheA4, HheA5, HheB, HheB2, HheBx, HheC, HheGx (group 4a), preferably from protein sequences or coding DNA sequences of HheA, HheA2, HheA3, HheA4, HheA5, HheB, HheB2, HheC (group 3a), more preferred from protein sequences or coding DNA sequences of HheA, HheA2, HheA4, HheB, HheB2, HheC (group 2a), most preferred from protein sequences or coding DNA sequences of HheA, HheA2, HheB, HheB2, HheC (group 1a).

In yet another possible aspect of the identification method, where at least one query biomolecule sequence of at least one halohydrin dehalogenase is used, i.e. steps a1) and a2) are used, the at least one query biomolecule sequence of at least one halohydrin dehalogenase is selected from protein sequences or coding DNA sequences of HheA3, HheA4, HheA5, HheA6, HheB3, HheB4, HheB5, HheB6, HheB7, HheBx, HheD, HheD2, HheD3, HheD4, HheD5, HheD6, HheD7, HheD8, HheD9, HheD10, HheD11, HheD12, HheD13, HheD14, HheD15, HheD16, HheD17, HheE, HheE2, HheE3, HheE4, HheE5, HheEx, HheEx2, HheEx3, HheF, HheG, HheGx (group 1b), preferably from protein sequences or coding DNA sequences of HheA3, HheA5, HheA6, HheB3, HheB4, HheB5, HheB6, HheB7, HheBx, HheD, HheD2, HheD3, HheD4, HheD5, HheD6, HheD7, HheD8, HheD9, HheD10, HheD11, HheD12, HheD13, HheD14, HheD15, HheD16, HheD17, HheE, HheE2, HheE3, HheE4, HheE5, HheEx, HheEx2, HheEx3, HheF, HheG, HheGx (group 2b), more preferred from protein sequences or coding DNA sequences of HheA6, HheB3, HheB4, HheB5, HheB6, HheB7, HheBx, HheD, HheD2, HheD3, HheD4, HheD5, HheD6, HheD7, HheD8, HheD9, HheD10, HheD11, HheD12, HheD13, HheD14, HheD15, HheD16, HheD17, HheE, HheE2, HheE3, HheE4, HheE5, HheEx, HheEx2, HheEx3, HheF, HheG, HheGx (group 3b), most preferred from protein sequences or coding DNA sequences of HheA6, HheB3, HheB4, HheB5, HheB6, HheB7, HheD, HheD2, HheD3, HheD4, HheD5, HheD6, HheD7, HheD8, HheD9, HheD10, HheD11, HheD12, HheD13, HheD14, HheD15, HheD16, HheD17, HheE, HheE2, HheE3, HheE4, HheE5, HheEx, HheEx2, HheEx3, HheF, HheG (group 4b).

Preferably, pre-target biomolecule sequences have a degree of sequence identity of at least 15%, preferably 20% to the at least one query biomolecule sequence, calculated on amino acid level. Also preferred is it when the pre-target biomolecule sequences have a degree of sequence identity of at most 80%, preferably of at most 70%, more preferably of at most 60% to the at least one query biomolecule sequence, calculated on amino acid level. Especially, if the sequences excluded as pre-target biomolecules or target biomolecules are used as query biomolecule, all sequences with more than 80% sequence identity to these query biomolecules, calculated on amino acid level, are excluded as pre-target biomolecules or target biomolecules.

In yet another possible aspect of the identification method pre- or target biomolecule sequences having more or coding for more than 350 amino acids, more preferred having more or coding for more than 280 amino acids, more preferred having more or coding for more than 262 amino acids are excluded.

It has to be noted that amino acids in each of the motifs (1)-(3) might be replaced without losing the HHDH function. This applies, for example, if the replacement-amino-acid has a similar structure, similar properties etc. as the replaced amino acid.

Regarding amino acid replacements in motif (1), it is possible that
- amino acid Threonine (T) can be replaced by amino acid Methionine (M) or by amino acid Serine (S) and/or
- amino acid Phenylalanine (F) or Tyrosine (Y) can be replaced by an amino acid selected from Isoleucine (I) or Leucine (L)
   and/or
- amino acid Glycine (G) can replaced by amino acid Serine (S).

The resulting motif (1) with replacement is thus:

"[TSM]-x(4)-[FYIL]-x-[GS]".

However, use of motif (1) without amino acid replacements is preferred.

Regarding amino acid replacements in motif (2), it is also possible that
- amino acid Tyrosine (Y) can be replaced by amino acid Proline (P)

The resulting motif (2) with replacement is thus:

"S-x(12)-[YP]-x(3)-[R]".

However, use of motif (2) without amino acid replacements is preferred.

### Use of target biomolecule sequences as HHDHs

The identified target biomolecule sequences are usable as HHDHs in the conversion from halohydrins to epoxides and/or in the ring-opening of epoxides to β-substituted alcohols. According to the invention, a biomolecule having the protein sequence or coding DNA sequence of HheD3 having NCBI, GenBank accession number ABM93639 is used as halohydrin dehalogenase.

### Conversion of halohydrins to epoxides

In detail, the biomolecule having the protein sequence or coding DNA sequence of HHeD3 having NCBI, GenBank accession number ABM93639 is used as halohydrin dehalogenase in the conversion of at least a part of the amount of a halohydrin of formula (I) into an epoxide of formula (II) 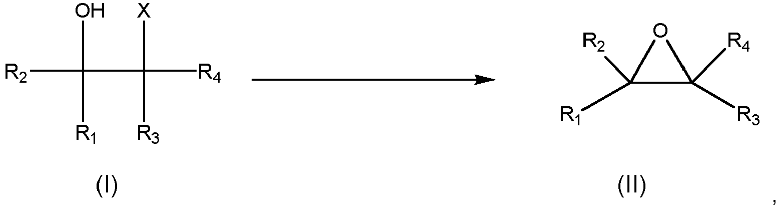 wherein
- X: is a halogen atom selected from Cl, Br, I;
- R₁, R₂, R₃, R₄: are independently hydrogen, halogen (F, Cl, Br, I) or substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl, or -C1-C3-alkyl-C(=O)-O-R₅ or -C1-C3-alkyl-O-R₅, with
- R₅: selected from hydrogen, C1-C6-alkyl, C2-C6-alkenyl, C2-C6-alkinyl, C1-C6-alkoxy, C5-C13-alkylaryl, C5-C13-arylalkyl, C5-C13-aryloxy, C5-C13-arylalkoxy, C5-C13-heteroalkyl, C5-C13-aryl, C5-C13-heteroaryl, C3-C12-cycloalkyl, C3-C12-cycloalkenyl, C6-C12-cycloalkinyl or C5-C13-cyclic heteroalkyl, wherein in case of more than one aliphatic or aromatic ring system the ring systems are condensed or separated and R₅ may have at least one further substituent R₆ with
- R₆: selected from hydrogen, hydroxyl, -C(=O)-NH₂, halogen (F, Cl, Br, I), -C1-C3-alkyl, -C(=)O-C1-C3-alkyl, -C(=)O-C1-C3-alkyl, -NH-C(=)O-C1-C3-alkyl, -C1-C3-alkyl-O-Cl-C3-alkyl, C2-C3-alkenyl, -O-C2-C3-alkenyl, C5-C7-cycloalkyl, C5-C7-cyclic heteroalkyl, C5-C7-aryl or C5-C7-heteroaryl, preferably selected from hydrogen and methyl,
and/or
two of R₁, R₂, R₃, R₄ together form a cyclic structure selected from C3-C12-cycloalkyl, C4-C12-cycloalkenyl, C6-C12-cycloalkinyl, C3-C12-cyclic heteroalkyl, C4-C12-cyclic heteroalkenyl or C6-C12-cyclic heteroalkinyl, preferably a C4-C8-cycloalkyl, or C4-C8-cycloalkenyl group, more preferred a C5-C7-cycloalkyl, or C5-C7-cycloalkenyl group, most preferred a C5-C6-cycloalkyl, or C5-C6-cycloalkenyl group wherein in case of more than one ring system the ring systems are condensed or separated, and wherein the number of C-atoms as indicated for the cyclic structure always includes the 2 C-atoms of the halohydrin structure bearing the OH and the X, and
wherein each cyclic group in R₁, R₂, R₃, R₄ or cyclic structure formed by two of R₁, R₂, R₃, R₄ optionally has at least one further substituent different from hydrogen, preferably selected from R6, most preferred halogen (F, Cl, Br, I) or C1-C3-alkyl. This optional at least one further substituent can also be named as substituent R₇, which is especially (R₇)ₙ with n being zero or an integer between 1 and 4, preferably n = 0, 1 or 2.

The terms alkene/alkenyl, cylcoalkene/cylcoalkenyl refer to hydrocarbons containing at least one carbon-carbon double bond. The terms alkine/alkinyl, cylcoalkine/cylcoalkinyl refer to hydrocarbons containing at least one carbon-carbon triple bond.

Regarding R₁, R₂, R₃, R₄: each of R₁, R₂, R₃, R₄, which does not form a cyclic structure with another of R₁, R₂, R₃, R₄, i.e. each of these residues R₁, R₂, R₃, R₄, which is not is covalently bounded to another of these residues R₁, R₂, R₃, R₄, may have at least one carbon atom replaced by a hetero atom. In other words: each R₁, R₂, R₃, R₄ being a hydrocarbon, i.e. substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl may have at least one carbon atom replaced by a hetero atom.

Overall, i.e. with respect to any of the residues R₁-R₇: The wording "hetero" or "hetero atom" preferably means a hetero atom selected from nitrogen (N), sulfur (S), oxygen (O), selen (Se) or phosphor (P), more preferably a hetero atom selected from N, S or O.

Regarding all substances of formula (I), the C-atoms bearing the OH and X are always sp³ hybridized. Simultaneously, the C-atoms forming the epoxide ring in formula (II) are also always sp³ hybridized.

The halohydrin of formula (I) is preferably selected from 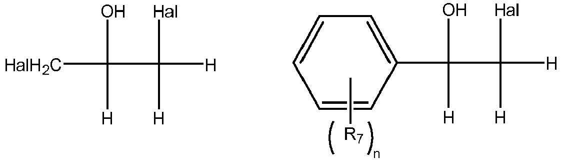 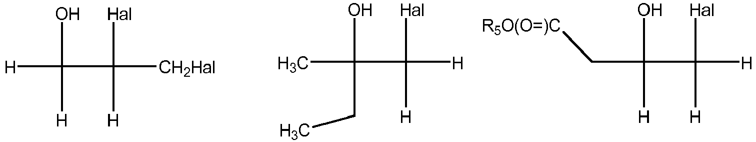 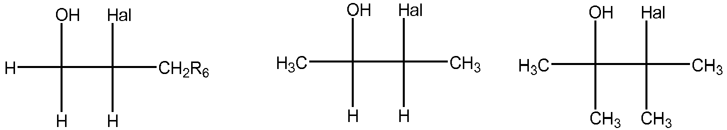 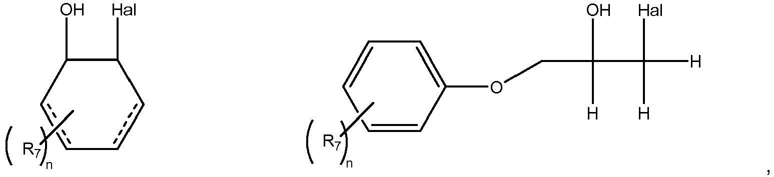 wherein Hal is Cl or Br and is in case of more than one Hal preferably identical per substance, and R7 is selected from halogen (F, Cl, Br, I) or C1-C3-alkyl and each ----- independently represents a double or single bond and n is zero or an integer between 1 and 4, preferably 0, 1 or 2.

### Ring-opening of epoxides to alcohols

The at least one biomolecule having the protein sequence or coding DNA sequence of HHeD3 having NCBI, GenBank accession number ABM93639 is also used as halohydrin dehalogenase in the conversion of at least a part of an epoxide of formula (II) into an alcohol of formula (III) 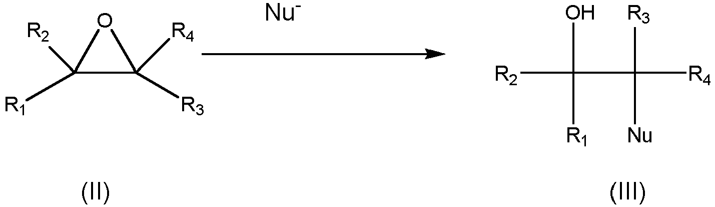 wherein
- R₁, R₂, R₃, R₄: are independently hydrogen, halogen (F, Cl, Br, I) or substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl, or -C1-C3-alkyl-C(=O)-O-R₅ or -C1-C3-alkyl-O-R₅, with
- R₅: selected from hydrogen, C1-C6-alkyl, C2-C6-alkenyl, C2-C6-alkinyl, C1-C6-alkoxy, C5-C13-alkylaryl, C5-C13-arylalkyl, C5-C13-aryloxy, C5-C13-arylalkoxy, C5-C13-heteroalkyl, C5-C13-aryl, C5-C13-heteroaryl, C3-C12-cycloalkyl, C3-C12-cycloalkenyl, C6-C12-cycloalkinyl or C5-C13-cyclic heteroalkyl, wherein in case of more than one aliphatic or aromatic ring system the ring systems are condensed or separated and R₅ may have at least one further substituent R₆ with
- R₆: selected from hydrogen, hydroxyl, -C(=O)-NH₂, halogen (F, Cl, Br, I), -C1-C3-alkyl, -C(=)O-C1-C3-alkyl, -C(=)O-C1-C3-alkyl, -NH-C(=)O-C1-C3-alkyl, -C1-C3-alkyl-O-C1-C3-alkyl, C2-C3-alkenyl, -O-C2-C3-alkenyl, C5-C7-cycloalkyl, C5-C7-cyclic heteroalkyl, C5-C7-aryl or C5-C7-heteroaryl, preferably selected from hydrogen and methyl,
and/or
two of R₁, R₂, R₃, R₄ together form a cyclic structure selected from C3-C12-cycloalkyl, C4-C12-cycloalkenyl, C6-C12-cycloalkinyl, C3-C12-cyclic heteroalkyl, C4-C12-cyclic heteroalkenyl or C6-C12-cyclic heteroalkinyl, preferably a C4-C8-cycloalkyl, or C4-C8-cycloalkenyl group, more preferred a C5-C7-cycloalkyl, or C5-C7-cycloalkenyl group, most preferred a C5-C6-cycloalkyl, or C5-C6-cycloalkenyl group wherein in case of more than one ring system the ring systems are condensed or separated, and wherein the number of C-atoms as indicated for the cyclic structure always includes the 2 C-atoms of the halohydrin structure bearing the OH and the X, and
wherein each cyclic group in R₁, R₂, R₃, R₄ or cyclic structure formed by two of R₁, R₂, R₃, R₄ optionally has at least one further substituent different from hydrogen, preferably selected from R6, most preferred halogen (F, Cl, Br, I) or C1-C3-alkyl. This optional at least one further substituent can also be named as substituent R₇, which is especially (R₇)ₙ with n being zero or an integer between 1 and 4, preferably n = 0, 1 or 2.
   Nu is selected from R'NO_{2,} N₃, NO₂, SCN, OCN, CN, halogen (F, Cl, Br, I, especially Cl, Br, I) and R"CO₂, wherein
   R' is selected from C1-C4-alkyl, C1-C4-alkenyl, preferably CH₃,
   R" is selected from hydrogen, C1-C4-alkyl, C1-C4-alkenyl, preferably hydrogen,
   and Nu⁻ is an anion of Nu.

In one special embodiment, Nu is selected from N₃, NO₂, SCN, OCN, CN, halogen (CI, Br, I) and R"CO₂, i.e. R'NO₂ is excluded. Preferably, the hydrocarbons in R' and/or in R" are linear/unbranched.

The terms alkene/alkenyl, cylcoalkene/cylcoalkenyl refer to hydrocarbons containing at least one carbon-carbon double bond as already explained above. The terms alkine/alkinyl, cylcoalkine/cylcoalkinyl refer to hydrocarbons containing at least one carbon-carbon triple bond.

Regarding R₁, R₂, R₃, R₄: each of R₁, R₂, R₃, R₄, which does not form a cyclic structure with another of R₁, R₂, R₃, R₄, i.e. each of these residues R₁, R₂, R₃, R₄, which is not is covalently bounded to another of these residues R₁, R₂, R₃, R₄, may have at least one carbon atom replaced by a hetero atom. In other words:, each R₁, R₂, R₃, R₄ being a hydrocarbon, i.e. substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl may have at least one carbon atom replaced by a hetero atom.

Overall, i.e. with respect to any of the residues R₁-R₇: The wording "hetero" or "hetero atom" preferably means a hetero atom selected from nitrogen (N), sulfur (S), oxygen (O), selen (Se) or phosphor (P), more preferably a hetero atom selected from N, S or O.

Regarding all substances of formula (II), the C-atoms forming the epoxide ring are always sp³ hybridized. Additionally, the C-atoms bearing the OH and Nu in formula (III) are always sp³ hybridized.

The epoxide of formula (II) is preferably selected from 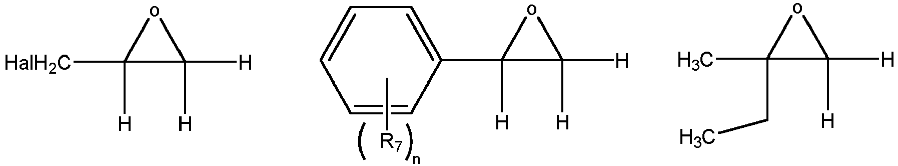 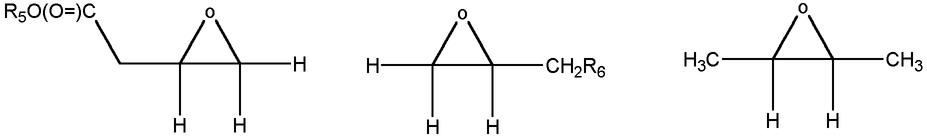 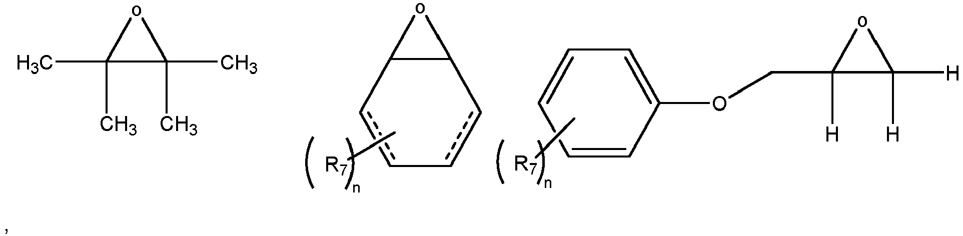 wherein Hal is Cl or Br and is in case of more than one Hal preferably identical per substance, and R7 is selected from hydrogen, halogen (F, Cl, Br, I) or C1-C3-alkyl and each ----- independently represents a double or single bond and n is zero or an integer between 1 and 4, preferably 0, 1 or 2.

In each formula (I), (II) and/or (III) at least two of R1, R2, R3, R4 are preferably hydrogen or-CH₃, more preferred one of R1, R2 and one of R3, R4 are hydrogen. This applies to the separate reactions described above and to the combined reaction explained below.

### Combined reaction

In a preferred embodiment of the invention, the biomolecule having protein sequence or coding DNA sequence of HHeD3 having NCBI, GenBank accession number ABM93639 is used as halohydrin dehalogenase in the conversion of at least a part of the amount of halohydrin of formula (I) into an epoxide of formula (II) and in the conversion of at least a part of the epoxide of formula (II) into an alcohol of formula (III). Preferably, this is done as one-vessel-reaction, one-pot synthesis, meaning that the initial substance of formula (I) is subjected to successive reactions, finally resulting in substances of formula (III) in just one reactor.

Regarding preferred embodiments of (I) and (III) in the combined reaction, the same applies as described above with respect to the separate reactions.

In possible variants of the conversion of at least a part of the amount of halohydrin of formula (I) into an epoxide of formula (II) and/or in the conversion of at least a part of an epoxide of formula (II) into an alcohol of formula (III), i.e. in separate reactions of (I) to (II) or of (II) to (III) or in a combined reaction of (I) to (III), the at least one biomolecule is a protein selected from HheA6, HheB3, HheB4, HheB5, HheB6, HheB7, HheD, HheD2, HheD3, HheD4, HheD5, HheD6, HheD7, HheD8, HheD9, HheD10, HheD11, HheD12, HheD13, HheD14, HheD15, HheD16, HheD17, HheE, HheE2, HheE3, HheE4, HheE5, HheEx, HheEx2, HheEx3, HheF, HheG, wherein only HHeD3 is covered by the present invention.

### Detailed Description of the invention

Below, the invention and its development are described in more detail.

### Sequence characteristics of HHDHs

First, in order to identify novel HHDH enzymes, all known HHDH sequences were inspected for distinctive residues which distinguish HHDHs from other SDR enzymes. All known HHDHs possess a catalytic triad of Ser-Tyr-Arg (SYR) which aligns with the catalytic residues Ser-Tyr-Lys (SYK) present in SDR sequences. Thus, the presence of Arg in the HHDH catalytic triad can be used as an initial criterion to filter for putative novel HHDHs from other SDR sequences. In addition, the crystal structures of HheA2 (23) and HheC (24) show that both HHDHs possess a spacious anion binding pocket which is formed in part by residues that align with residues of a Gly-rich motif responsible for nucleotide cofactor binding in Rossman-fold enzymes such as SDR enzymes. Specifically, the large residue F12 in both HheA2 and HheC is essential for the formation of the HHDH anion binding pocket and replaces a small Gly or Ala in the "T-G-x(3)-[GA]-x-G" nucleotide binding motif of classical SDR enzymes (22-24). In known enzymes HheB/B2, a Tyr residue aligns with the Phe of the other known HHDHs. Thus, in all known HHDH enzymes, the large aromatic amino acids Phe (F) or Tyr (Y) disturb the commonly observed Gly-rich cofactor binding motif of SDR enzymes. In consequence, the inventors claim that the presence of both the HHDH catalytic triad (resembled by motif (2), see below) and the aromatic Phe/Tyr (included in motif (1), see below) indicates sequences with HHDH activity. Especially the definition of motif (1), with an aromatic amino acid instead of the normal Gly-rich motif, is new and has not been made before. In combination with motif (2), motif (1) is sufficient to discriminate true HHDH sequences from unrelated SDR sequences.

### Database mining

To identify novel HHDH sequences, BLASTp searches were initiated to collect homologous sequences from GenBank which could then be assessed for the presence of conserved HHDH sequence features.

From the GenBank collection of non-redundant sequences (nr) and non-redundant environmental sequences (env_nr), a lot of novel HHDH sequences (Table 1) contained the catalytic triad of known HHDHs in combination with the aromatic Phe or Tyr specific for known HHDH enzymes. For the obtained novel HHDH sequences, no reports on their activity can be retrieved from the associated GenBank records as these records are mostly annotated as "SDR enzyme" or "oxidoreductase" but never as HHDH enzyme.

**TABLE 1 Sources and accession numbers of previously known HHDHs with proven activity (*), of previously as HHDH-named enzymes without proof of HHDH activity or of sequences with high sequence identity compared to known HHDHs (**) and novel HHDHs**

| **HHDH** | **Organism or source** | **Accession** (NCBI, GenBank) |
|---|---|---|
| HheA* | Corynebacterium sp. | BAA14361 |
| HheA2* | Arthrobacter sp. AD2 | AAK92100 |
| HheA3** | Parvibaculum Iavamentivorans DS-1 | ABS64560 |
| HheA4** | Arthrobacter sp. JBH1 | AFI98638 |
| HheA5** | Tistrella mobilis KA081020-065 | AFK51877 |
| HheA6 | bacterium Ec32 | CDO61292 |
| HheB* | Corynebacterium sp. | BAA14362 |
| HheB2* | Mycobacterium sp. GP1 | AAK73175 |
| HheB3 | marine metagenome | SEQ ID NO.1 |
| HheB4 | marine metagenome | EBP61646 |
| HheB5 | marine metagenome | SEQ ID NO.2 |
| HheB6 | marine metagenome | SEQ ID NO.3 |
| HheB7 | marine metagenome | SEQ ID NO.4 |
| HheBx | uncultured marine bacterium Ant4E12 | SEQ ID NO.15 |
| HheC* | Agrobacterium tumefaciens | AAK92099 |
| HheD | Dechloromonas aromatica RCB | AAZ44846 |
| HheD2 | gamma proteobacterium HTCC2207 | EAS46473 |
| HheD3 | Methylibium petroleiphilum PM1 | ABM93639 |
| HheD4 | marine metagenome | SEQ ID NO.5 |
| HheD5 | Thauera sp. MZ1T | YP_002355872 |
| HheD6 | Marinobacter nanhaiticus D15-8W | ENO15189 |
| HheD7 | Thauera sp. 27 | ENO82779 |
| HheD8 | Thauera aminoaromatica S2 | ENO87252 |
| HheD9 | Thauera phenylacetica B4P | ENO98837 |
| HheD10 | Limnohabitans sp. Rim28 | SEQ ID NO.6 |
| HheD11 | Curvibacter Ianceolatus | SEQID NO.7 |
| HheD12 | Thiothrix disciformis | WP_020394200 |
| HheD13 | Pseudomonas pelagia | WP_022962804 |
| HheD14 | Betaproteobacteria bacterium | ESS13801 |
| HheD15 | Gammaproteobacteria bacterium | ETN91936 |
| HheD16 | candidatus Competibacter | CDI00977 |
| HheD17 | Methylibium sp. T29 | EWS52496 |
| HheE | marine metagenome | EBP63112 |
| HheE2 | marine metagenome | ECW41905 |
| HheE3 | marine metagenome | SEQ ID NO.8 |
| HheE4 | marine metagenome | SEQID NO.9 |
| HheE5 | gamma proteobacterium IMCC3088 | SEQID NO.10 |
| HheEx | marine gamma proteobacterium | SEQID NO.11 |
| HheEx2 | marine metagenome | SEQID NO.12 |
| HheEx3 | marine metagenome | SEQID NO.13 |
| HheF | uncultured bacterium | SEQID NO.14 |
| HheG | Ilumatobacter coccineus YM16-304 | BAN03849 |
| HheGx | Microbacterium yannicii | WP_019180129 |

| | | |
|---|---|---|
| The with ** indicated sequences in Table 1 are only named as HHDHs or are similar to previously known HHDHs (*) but their activity was never proven (for more details, see above). | | |

Several of the novel sequences (SEQ ID NO. 11-13 and 15) in Table 1 correspond/are similar to sequences listed in databases, wherein the database listed sequences miss amino acids at the N- or C- terminus, which were supplemented here by the inventors. Regarding the novel sequences SEQ ID NO. 1-10 and 14, the sequences listed in databases contain a surplus of amino acids, which were deleted by the inventors. For these database listed sequences, no reports on their activity can be retrieved from the associated GenBank records as these records are mostly annotated as "SDR enzyme" or "oxidoreductase". Only the database listed sequence corresponding to HheBx (GenBank: ABC25271) is annotated as HHDH but - due to the missing sequence pieces - cannot work as HHDH. The corresponding accessions of the database listed sequences are given in Table 2 below:

**TABLE 2 Corresponding accessions of listed but incomplete or too long sequences**

| **HHDH** | **Accession (NCBI, GenBank)** | **SEQ ID NO.** |
|---|---|---|
| HheB3 | EBL02020 | 1 |
| HheB5 | ECR06649 | 2 |
| HheB6 | EDB56284 | 3 |
| HheB7 | EDD65701 | 4 |
| HheBx | ABC25271 | 15 |
| HheD4 | ECY18578 | 5 |
| HheD10 | WP_019427705 | 6 |
| HheD11 | WP_019574066 | 7 |
| HheE3 | EDF62577 | 8 |
| HheE4 | EDH34310 | 9 |
| HheE5 | EGG28524 | 10 |
| HheEx | EAW33023 | 11 |
| HheEx2 | EBC61576 | 12 |
| HheEx3 | EBF34592 | 13 |
| HheF | BAH89601 | 14 |

Especially HheBx is only functional with an amino acid sequence according to SEQ ID NO. 15. The database listed variation ABC25271 is not functional since motif (1) is missing.

### Description of Figures

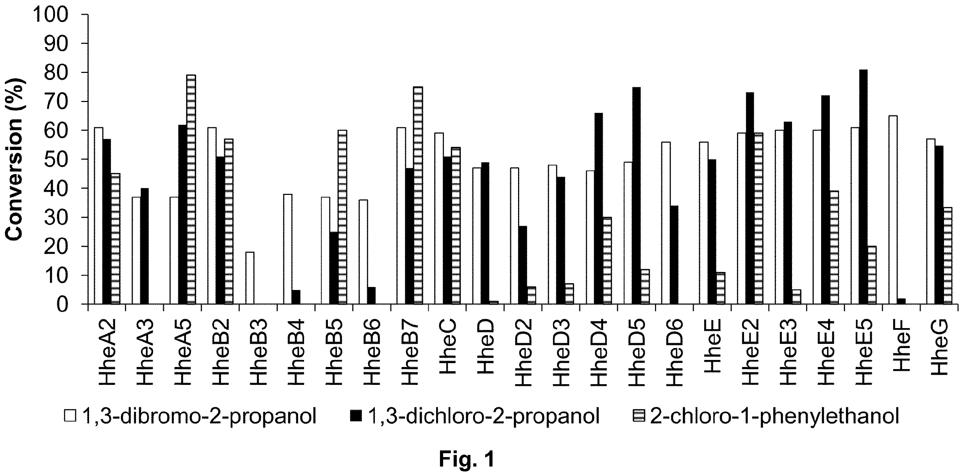

- **FIG 1**: Conversions of different haloalcohols with cell free extracts from recombinant HHDH expression. Conversions are compared for the formation of corresponding epoxides from 1,3-dibromo-2-propanol (white), 1,3-dichloro-2-propanol (black) and 2-chlorophenylethanol (lines) after deduction of background activities from empty vector controls.

### Experimental verification of HHDH activity

In order to investigate if the identified sequences indeed represent enzymes with true HHDH activity, a selection of 19 codon-optimized synthetic genes was ordered for heterologous expression of HheA3, HheA5, HheB3, HheB4, HheB5, HheB6, HheB7, HheD, HheD2, HheD3, HheD4, HheD5, HheE, HheE2, HheE3, HheE4, HheE5, HheF or HheG in *E. coli.*

To confirm HHDH activity, all 19 synthetic HHDH genes were cloned into the well-established pET-28a expression vector. Heterologous expression of soluble enzyme was optimized for each HHDH by varying parameters such as the expression host [*E. coli* BL21(DE3) or C43(DE3)] or expression temperature (20, 30 or 37°C). The optimization of expression conditions yielded visible bands of soluble enzyme for most HHDHs after Coomassie staining of polyacrylamide gels which were not present in empty vector controls.

To assess whether these recombinant enzymes possess true HHDH activity, a colorimetric activity assay for halide release from chloroalcohols 1,3-dichloro-2-propanol, 2-chlorophenylethanol and 1,3-dibromo-2-propanol was performed with cell-free extract (CFE) of cells containing the recombinantly expressed enzymes. Afterwards, gas chromatography analysis was used to confirm formation of the corresponding epoxides. For all selected HHDHs, halide release as well as epoxide formation could be detected for at least one of the substrates. Fig. 1 summarizes the obtained conversions of the different HHDH-containing CFEs (as calculated from the amount of formed epoxide after deduction of empty vector controls). Known HHDHs, HheA2, HheB2 and HheC, were included in the measurements for comparison.

### Fingerprinting for exclusive recovery of HHDH sequences

After it was confirmed that the approach specifically identifies sequences which exhibit true HHDH activity, it was further tried to optimize the search routine to identify even more distantly related sequences and, at the same time, simplify the overall procedure.

In the initial BLASTp searches for novel HHDH sequences, the large majority of results was dominated by SDR sequences and thus distantly related HHDHs might have been overlooked. To circumvent this, PHI-BLAST can be used to detect more distantly related sequences which match a user-defined pattern (25). To recognize such pattern, the MAFFT alignment of all known and novel HHDH sequences was inspected for the presence of conserved motifs.

As expected from the outlined identification protocol, all identified HHDHs necessarily possessed the Ser-Tyr-Arg catalytic triad resembled by pattern "S-x(12)-Y-x(3)-R". In addition, the conserved aromatic Phe or Tyr is part of a HHDH-specific pattern "T-x(4)-[FY]-x-G" which contrasts the SDR "T-G-x(3)-[GA]-x-G" motif. Consequently, any of the HHDH-specific patterns can serve as seed pattern for PHI-BLAST searches.

The new (HHDH amino acid sequence) motif (1) is thus

(1) "T-x(4)-[FY]-x-G"

and the new (HHDH amino acid sequence) motif (2) is thus

(2) "S-x(12)-Y-x(3)-R",

With either pattern as seed for PHI-BLAST searches, sequences of each phylogenetic HHDH type, namely HheB, HheC, HheD, HheE, HheF and HheG, were used to query the nr database. Within these results, again SDR enzymes were included but also all experimentally verified HHDHs which had been identified earlier. Thus, each pattern alone is sufficient to recover the available HHDH sequences independent of the query sequence.

Increasing the E cut-off value (up to 1000) and using any of the other HHDH sequences as query, no further HHDH sequence could be identified.

Although each pattern alone greatly reduces the total number of sequences to be assessed for the complementary sequence feature, still, SDR sequences were always included in all of the results. For the specific retrieval of only HHDH sequences, it is possible to combine both patterns to identify sequences which must agree in both of the aforementioned sequence characteristics. As both patterns are separated by 93 to 131 residues in the experimentally verified HHDHs, both motifs can be combined in pattern "T-x(4)-[FY]-x-G-x(93,131)-S-x(12)-Y-x(3)-R", or, more general "T-x(4)-[FY]-x-G-x(1,250)-S-x(12)-Y-x(3)-R".

Now, using the combined pattern with any HHDH sequence as PHI-BLAST query, only the novel HHDHs (Table 1) are retrieved as relevant hits (E value <0.1). Varying the gap length between 1 and 250 residues does not recover any additional relevant sequence (with an E value <0.1) with both HHDH sequence features. Querying the updated env_nr database with separate or combined patterns did not result in the recovery of additional putative novel HHDH sequences.

Furthermore, the known and novel HHDHs as well as other SDR enzymes possess a conserved motive (3) of "G-x(6)-G-G" which is crucial for their oligomerization. As some reports suggest that at least HheC is inactivate if formation of the tetramer is inhibited, this third motif might be helpful in discerning active HHDH enzymes.

Thus, a third motif (3) was identified:

(3) "G-x(6)-G-G"

As pattern (3) is separated by 76 to 84 residues in the experimentally verified HHDHs, all three motifs can be combined in pattern "T-x(4)-[FY]-x-G-x(93,131)-S-x(12)-Y-x(3)-R-x(76,84)-G-x(6)-G-G", or, more general "T-x(4)-[FY]-x-G-x(1,250)-S-x(12)-Y-x(3)-R-x(1,250)-G-x(6)-G-G".

In summary, it was possible to greatly reduce the to-be-analyzed sequences from homology searches by making use of restrictive, HHDH-specific sequence patterns especially "T-x(4)-[FY]-x-G" and/or "S-x(12)-Y-x(3)-R". The combination of patterns (1), (2) and (3), most preferably (1) and (2), is able to discern non-HHDH sequences from homology search results (E ≥ 0.1) and is in consequence highly useful for the precise identification of HHDH sequences present in public databases from the vast number of similar but different SDR sequences.

### Material and Methods

**Database mining:** The sequences of HheA, HheB and HheC (Table 1) were used as queries for BLASTp searches (26) of the nr and env_nr database of GenBank (release 200) (27). For each query, 20,000 sequences were retrieved and used together with HheA, HheB and HheC for the construction of multiple sequence alignments (MSAs) with MAFFT (FFT-NS-2) (28). In the resulting alignments, sequences were dismissed if they possessed the typical Ser-Tyr-Lys catalytic residues of SDR enzymes which aligned with the Ser-Tyr-Arg catalytic triad of HheA/HheA2 (S134-Y148-R151), HheB/HheB2 (S127-Y139-R143) and HheC (S132-Y145-R149). After removal of those putative SDR sequences, the reduced sequence pool of remaining sequences was re-aligned using MAFFT (FFT-NS-2). Afterwards, only sequences with a catalytic triad of Ser-Tyr-Arg also present in known HHDH enzymes were selected for construction of a new MAFFT (L-INS-i) (29) MSA. From the latter alignment, only sequences were considered to be putative novel HHDHs if they possessed an aromatic Phe or Tyr which aligned with F12, Y27, and F12 from HheA/HheA2, HheB/HheB2, and HheC, respectively. All novel HHDH sequences were used as queries in subsequent search routines which consisted of BLASTp searches, construction of MSAs and their inspection for the presence of the typical HHDH catalytic triad in combination with the conserved aromatic residue close to the N-terminus as outlined above. This strategy was continued until no additional novel HHDH sequences could be identified in the nr and env_nr database.
**Gene synthesis and cloning:** Synthetic genes coding for 19 HHDHs (HheA3, HheA5, HheB3, HheB4, HheB5, HheB6, HheB7, HheD, HheD2, HheD3, HheD4, HheD5, HheE, HheE2, HheE3, HheE4, HheE5, HheF, HheG) were ordered from Life Technologies (Darmstadt, Germany) after back translation of the curated protein sequences and codon optimization for *E. coli* with GeneOptimizer (30). The synthetic genes were excised from received plasmids by restriction with Ndel and either Hindlll or Xhol followed by ligation with T4 DNA ligase (all DNA modifying enzymes from New England Biolabs, Frankfurt, Germany) into linearized pET-28a (Merck, Darmstadt, Germany). After transformation into *E. coli* DH5α (Life Technologies), recombinant plasmid DNA was prepared with the NucleoSpin Plasmid kit (Macherey Nagel, Düren, Germany) and was sent for sequencing at GATC Biotech (Konstanz, Germany).
**Expression and activity assays:** Plasmids containing the different HHDH genes were either transformed into *E. coli* BL21(DE3) (Life Technologies), or C43(DE3) (Lucigen Corporation, Middleton, WI, USA) for heterologous protein expression. Each 20 mL TB medium containing 50 mg L⁻¹ kanamycin and 0.2 mM IPTG were inoculated with 10% v/v of a respective *E. coli* preculture and incubated at 20, 30 or 37°C for 7 to 24 h. Afterwards, cultures were centrifuged and pellets were stored at -20°C. For comparison, known enzymes HheA2, HheB2 and HheC were recombinantly expressed in *E. coli* Top10 (Life Technologies) from vector pBAD (Life Technologies) in TB medium with 100 mg L⁻¹ ampicillin and 0.02% L-arabinose. To prepare cell free extracts (CFEs), cell pellets were resuspended in 1.2 mL 25 mM Tris·SO₄ buffer, pH 8.0, and disrupted by sonication. After centrifugation, each 200 µL of these CFEs were added to 600 µL 25 mM Tris·SO₄ buffer containing one of the substrates 1,3-dichloro-2-propanol, 2-chlorophenylethanol or 1,3-dibromo-2-propanol in 10 mM final concentration for activity assays. Reactions were incubated at 30°C and each 50 µL samples were taken after 5, 20 and 60 min incubation to monitor dehalogenase activity using the halide release assay as described elsewhere (31). In addition after 60 min, remaining 650 µL per reaction were extracted once with each 600 µL methyl *tert*-butyl ether. Organic extracts were dried over magnesium sulfate and analyzed on a GC2010 gas chromatograph (Shimadzu, Japan) equipped with a Supreme 5ms column (CS Chromatography Service, Germany). GC analysis of reactions containing 1,3-dichloro-2-propanol or 1,3-dibromo-2-propanol was carried out with a temperature gradient starting at 40°C for 1 min, heating with 10°C min⁻¹ to 120°C and afterwards with 20°C min⁻¹ to 300°C. In case of reactions containing 2-chlorophenylethanol, the temperature gradient started at 80°C for 1 min, heating with 10°C min⁻¹ to 160°C and finally with 20°C min⁻¹ to 300°C. Substrates eluted at 6.6 min (1,3-dichloro-2-propanol), 8.6 min (2-chlorophenylethanol) and 9.4 min (1,3-dibromo-2-propanol) whereas corresponding products were detected at 3.7 min (epichlorohydrin), 5.8 min (styrene oxide) and 4.8 min (epibromohydrin), respectively.
**HHDH fingerprinting:** Conserved residues were deduced from MAFFT alignments of either all HHDH or with homologous SDR sequences. Patterns "T-x(4)-[FY]-x-G" (motif 1), "S-x(12)-Y-x(3)-R" (motif 2), or combinations thereof were used as seeds in PHI-BLAST (25) searches of the GenBank nr and env_nr database (release 200). Here, HheB, HheC, HheD, HheE, HheF, and HheG were used as queries with an E threshold of 10. All hits were aligned with MAFFT (FFT-NS-2) and the resulting MSA was inspected for sequences with a HHDH catalytic triad (motif 2) in combination with the aromatic Phe or Tyr (within motif 1) present in known HHDH enzymes as outlined above.

### References

1. Janssen DB, Majeric-Elenkov M, Hasnaoui G, Hauer B, Lutje Spelberg JH. 2006. Enantioselective formation and ring-opening of epoxides catalysed by halohydrin dehalogenases. Biochem. Soc. Trans. 34:291-295.
2. Schallmey M, Floor RJ, Szymanski W, Janssen DB. 2012. 7.8 Hydrolysis and Reverse Hydrolysis: Halohydrin Dehalogenases, p. 143-155. In Editors-in-Chief: Erick M. Carreira, Hisashi Yamamoto (eds.), Comprehensive Chirality. Elsevier, Amsterdam.
3. You Z-Y, Liu Z-Q, Zheng Y-G. 2012. Properties and biotechnological applications of halohydrin dehalogenases: current state and future perspectives. Appl. Microbiol. Biotechnol. 97:9-21.
4. Nakamura T, Nagasawa T, Yu F, Watanabe I, Yamada H. 1992. Resolution and some properties of enzymes involved in enantioselective transformation of 1,3-dichloro-2-propanol to (R)-3-chloro-1,2-propanediol by Corynebacterium sp. strain N-1074. J Bacteriol 174:7613-7619.
5. Lutje Spelberg JH, van Hylckama Vlieg JET, Bosma T, Kellogg RM, Janssen DB. 1999. A tandem enzyme reaction to produce optically active halohydrins, epoxides and diols. Tetrahedron Asymmetry 10:2863-2870.
6. Haak RM, Tarabiono C, Janssen DB, Minnaard AJ, de Vries JG, Feringa BL. 2007. Synthesis of enantiopure chloroalcohols by enzymatic kinetic resolution. Org. Biomol. Chem. 5:318-323.
7. Haak RM, Berthiol F, Jerphagnon T, Gayet AJA, Tarabiono C, Postema CP, Ritleng V, Pfeffer M, Janssen DB, Minnaard AJ, Feringa BL, de Vries JG. 2008. Dynamic Kinetic Resolution of Racemic β-Haloalcohols: Direct Access to Enantioenriched Epoxides. J. Am. Chem. Soc. 130:13508-13509.
8. Jin H-X, Hu Z-C, Liu Z-Q, Zheng Y-G. 2012. Nitrite-mediated synthesis of chiral epichlorohydrin using halohydrin dehalogenase from Agrobacterium radiobacter AD1. Biotechnol. Appl. Biochem. 59:170-177.
9. Majeric Elenkov M, Primožič I, Hrenar T, Smolko A, Dokli I, Salopek-Sondi B, Tang L. 2012. Catalytic activity of halohydrin dehalogenases towards spiroepoxides. Org. Biomol. Chem. 10:5063-5072.
10. Hasnaoui-Dijoux G, Majeric Elenkov M, Lutje Spelberg JH, Hauer B, Janssen DB. 2008. Catalytic Promiscuity of Halohydrin Dehalogenase and its Application in Enantioselective Epoxide Ring Opening. ChemBioChem 9:1048-1051.
11. Nakamura T, Nagasawa T, Yu F, Watanabe I, Yamada H. 1994. A new enzymatic synthesis of (R)-gamma-chloro-beta-hydroxybutyronitrile. Tetrahedron 50:11821-11826.
12. Lutje Spelberg JH, Tang L, Kellogg RM, Janssen DB. 2004. Enzymatic dynamic kinetic resolution of epihalohydrins. Tetrahedron Asymmetry 15:1095-1102.
13. Fox RJ, Davis SC, Mundorff EC, Newman LM, Gavrilovic V, Ma SK, Chung LM, Ching C, Tam S, Muley S, Grate J, Gruber J, Whitman JC, Sheldon RA, Huisman GW. 2007. Improving catalytic function by ProSAR-driven enzyme evolution. Nat. Biotechnol. 25:338-344.
14. Majeric Elenkov M, Hauer B, Janssen DB. 2006. Enantioselective Ring Opening of Epoxides with Cyanide Catalysed by Halohydrin Dehalogenases: A New Approach to Non-Racemic β-Hydroxy Nitriles. Adv. Synth. Catal. 348:579-585.
15. Majeric Elenkov M, Hoeffken HW, Tang L, Hauer B, Janssen DB. 2007. Enzyme-Catalyzed Nucleophilic Ring Opening of Epoxides for the Preparation of Enantiopure Tertiary Alcohols. Adv. Synth. Catal. 349:2279-2285.
16. Fuchs M, Simeo Y, Ueberbacher BT, Mautner B, Netscher T, Faber K. 2009. Enantiocomplementary Chemoenzymatic Asymmetric Synthesis of (R)- and (S)-Chromanemethanol. Eur. J. Org. Chem. 2009:833-840.
17. Molinaro C, Guilbault A-A, Kosjek B. 2010. Resolution of 2,2-Disubstituted Epoxides via Biocatalytic Azidolysis. Org. Lett. 12:3772-3775.
18. Yu F, Nakamura T, Mizunashi W, Watanabe I. 1994. Cloning of two halohydrin hydrogen-halide-lyase genes of Corynebacterium sp. strain N-1074 and structural comparison of the genes and gene products. Biosci. Biotechnol. Biochem. 58:1451-1457.
19. Van Hylckama Vlieg JET, Tang L, Lutje Spelberg JH, Smilda T, Poelarends GJ, Bosma T, van Merode AEJ, Fraaije MW, Janssen DB. 2001. Halohydrin Dehalogenases Are Structurally and Mechanistically Related to Short-Chain Dehydrogenases/Reductases. J. Bacteriol. 183:5058-5066.
20. Higgins TP, Hope SJ, Effendi AJ, Dawson S, Dancer BN. 2005. Biochemical and molecular characterisation of the 2,3-dichloro-1-propanol dehalogenase and stereospecific haloalkanoic dehalogenases from a versatile Agrobacterium sp. Biodegradation 16:485-492.
21. De Jong RM, Dijkstra BW. 2003. Structure and mechanism of bacterial dehalogenases: different ways to cleave a carbon-halogen bond. Curr. Opin. Struct. Biol. 13:722-730.
22. Kavanagh KL, Jörnvall H, Persson B, Oppermann U. 2008. Medium- and short-chain dehydrogenase/reductase gene and protein families. Cell. Mol. Life Sci. 65:3895-3906.
23. De Jong RM, Kalk KH, Tang L, Janssen DB, Dijkstra BW. 2006. The X-Ray Structure of the Haloalcohol Dehalogenase HheA from Arthrobacter sp. Strain AD2: Insight into Enantioselectivity and Halide Binding in the Haloalcohol Dehalogenase Family. J. Bacteriol. 188:4051-4056.
24. De Jong RM, Tiesinga JJW, Rozeboom HJ, Kalk KH, Tang L, Janssen DB, Dijkstra BW. 2003. Structure and mechanism of a bacterial haloalcohol dehalogenase: a new variation of the short-chain dehydrogenase/reductase fold without an NAD(P)H binding site. EMBO J. 22:4933-4944.
25. Zhang Z, Miller W, Schäffer AA, Madden TL, Lipman DJ, Koonin EV, Altschul SF. 1998. Protein sequence similarity searches using patterns as seeds. Nucleic Acids Res. 26:3986-3990.
26. Altschul SF, Madden TL, Schäffer AA, Zhang J, Zhang Z, Miller W, Lipman DJ. 1997. Gapped BLAST and PSI-BLAST: a new generation of protein database search programs. Nucleic Acids Res. 25:3389 -3402.
27. Benson DA, Cavanaugh M, Clark K, Karsch-Mizrachi I, Lipman DJ, Ostell J, Sayers EW. 2013. GenBank. Nucleic Acids Res. 41:D36-D42.
28. Katoh K, Misawa K, Kuma K, Miyata T. 2002. MAFFT: a novel method for rapid multiple sequence alignment based on fast Fourier transform. Nucleic Acids Res. 30:3059-3066.
29. Katoh K, Kuma K, Toh H, Miyata T. 2005. MAFFT version 5: improvement in accuracy of multiple sequence alignment. Nucleic Acids Res. 33:511 -518.
30. Raab D, Graf M, Notka F, Schödl T, Wagner R. 2010. The GeneOptimizer Algorithm: using a sliding window approach to cope with the vast sequence space in multiparameter DNA sequence optimization. Syst. Synth. Biol. 4:215-225.
31. Schallmey M, Floor RJ, Hauer B, Breuer M, Jekel PA, Wijma HJ, Dijkstra BW, Janssen DB. 2013. Biocatalytic and Structural Properties of a Highly Engineered Halohydrin Dehalogenase. ChemBioChem 14:870-881.
A Schallmey A, Schallmey M, Wardenga R, BlOspektrum, 2013, 19, 7, pp. 816 -817

## Claims

1. Use of a biomolecule having the protein sequence or coding DNA sequence of HheD3 having NCBI, GenBank accession number ABM93639 as halohydrin dehalogenase in the conversion of at least a part of the amount of a halohydrin of formula (I) into an epoxide of formula (II) 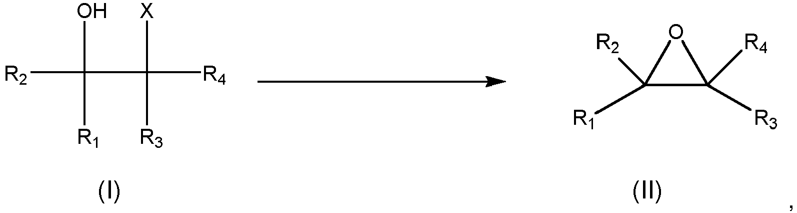 wherein
X is a halogen atom selected from Cl, Br, I;
R₁, R₂, R₃, R₄ are independently hydrogen, halogen (F, Cl, Br, I) or substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl, or
C1-C3-alkyl-C(=O)-O-R₅ or -C1-C3-alkyl-O-R₅, with
R₅ selected from hydrogen, C1-C6-alkyl, C2-C6-alkenyl, C2-C6-alkinyl, C1-C6-alkoxy, C5-C13-alkylaryl, C5-C13-arylalkyl, C5-C13-aryloxy, C5-C13-arylalkoxy, C5-C13-heteroalkyl, C5-C13-aryl, C5-C13-heteroaryl, C3-C12-cycloalkyl, C3-C12-cycloalkenyl, C6-C12-cycloalkinyl or C5-C13-cyclic heteroalkyl, wherein in case of more than one aliphatic or aromatic ring system the ring systems are condensed or separated and R₅ may have at least one further substituent R₆ with
R₆ selected from hydrogen, hydroxyl, -C(=O)-NH₂, halogen (F, Cl, Br, I), -C1-C3-alkyl, -C(=)O-Cl-C3-alkyl, -C(=)O-C1-C3-alkyl, -NH-C(=)O-C1-C3-alkyl, -C1-C3-alkyl-O-C1-C3-alkyl, C2-C3-alkenyl, -O-C2-C3-alkenyl, C5-C7-cycloalkyl, C5-C7- cyclic heteroalkyl, C5-C7-aryl or C5-C7-heteroaryl
and/or
two of R₁, R₂, R₃, R₄ together form a cyclic structure selected from C3-C12-cycloalkyl, C4-C12-cycloalkenyl, C6-C12-cycloalkinyl, C3-C12-cyclic heteroalkyl, C4-C12-cyclic heteroalkenyl or C6-C12-cyclic heteroalkinyl, preferably a C4-C8-cycloalkyl, or C4-C8-cycloalkenyl group, more preferred a C5-C7-cycloalkyl, or C5-C7-cycloalkenyl group, most preferred a C5-C6-cycloalkyl, or C5-C6-cycloalkenyl group wherein in case of more than one ring system the ring systems are condensed or separated, and
wherein the number of C-atoms as indicated for the cyclic structure always includes the 2 C-atoms of the halohydrin structure bearing the OH and the X, and
wherein each cyclic group in R₁, R₂, R₃, R₄ or cyclic structure formed by two of R₁, R₂, R₃, R₄ optionally has at least one further substituent different from hydrogen, preferably selected from R₆, most preferred halogen (F, Cl, Br, I) or C1-C3-alkyl (R₇).

2. Use of a biomolecule having the protein sequence or coding DNA sequence of HheD3 having NCBI, GenBank accession number ABM93639 as halohydrin dehalogenase in the conversion of at least a part of an epoxide of formula (II) into an alcohol of formula (III) 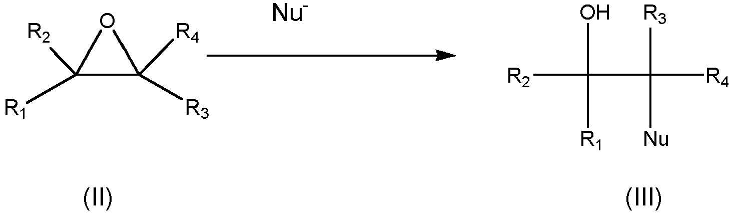 wherein
R₁, R₂, R₃, R₄ are independently hydrogen, halogen (F, Cl, Br, I) or substituted or unsubstituted C1-C12-alkyl, C2-C12-alkenyl, C2-C12-alkinyl, C1-C12-alkoxy, C3-C12-cycloalkyl, C3-C12-cycloalkenyl, C6-C12-cycloalkinyl or C5-C12-aryl, or
-C1-C3-alkyl-C(=O)-O-R₅ or -C1-C3-alkyl-O-R₅, with
R₅ selected from hydrogen, C1-C6-alkyl, C2-C6-alkenyl, C2-C6-alkinyl, C1-C6-alkoxy, C5-C13-alkylaryl, C5-C13-arylalkyl, C5-C13-aryloxy, C5-C13-arylalkoxy, C5-C13-heteroalkyl, C5-C13-aryl, C5-C13-heteroaryl, C3-C12-cycloalkyl, C3-C12-cycloalkenyl, C6-C12-cycloalkinyl or C5-C13-cyclic heteroalkyl, wherein in case of more than one aliphatic or aromatic ring system the ring systems are condensed or separated and R₅ may have at least one further substituent R₆ with
R₆ selected from hydrogen, hydroxyl, -C(=O)-NH₂, halogen (F, Cl, Br, I), -C1-C3-alkyl, -C(=)O-Cl-C3-alkyl, -C(=)O-C1-C3-alkyl, -NH-C(=)O-C1-C3-alkyl, -C1-C3-alkyl-O-C1-C3-alkyl, C2-C3-alkenyl, -O-C2-C3-alkenyl, C5-C7-cycloalkyl, C5-C7- cyclic heteroalkyl, C5-C7-aryl or C5-C7-heteroaryl
and/or
two of R₁, R₂, R₃, R₄ together form a cyclic structure selected from C3-C12-cycloalkyl, C4-C12-cycloalkenyl, C6-C12-cycloalkinyl, C3-C12-cyclic heteroalkyl, C4-C12-cyclic heteroalkenyl or C6-C12-cyclic heteroalkinyl, preferably a C4-C8-cycloalkyl, or C4-C8-cycloalkenyl group, more preferred a C5-C7-cycloalkyl, or C5-C7-cycloalkenyl group, most preferred a C5-C6-cycloalkyl, or C5-C6-cycloalkenyl group wherein in case of more than one ring system the ring systems are condensed or separated, and wherein the number of C-atoms as indicated for the cyclic structure always includes the 2 C-atoms of the halohydrin structure bearing the OH and the X, and
wherein each cyclic group in R₁, R₂, R₃, R₄ or cyclic structure formed by two of R₁, R₂, R₃, R₄ optionally has at least one further substituent different from hydrogen, preferably selected from R₆, most preferred halogen (F, Cl, Br, I) or C1-C3-alkyl (R₇) and
Nu is selected from R'NO₂ N₃, NO₂, SCN, OCN, CN, halogen (F, Cl, Br, I) and
R"CO₂, wherein
R' is selected from C1-C4-alkyl, -alkenyl, preferably CH₃,
R" is selected from hydrogen, C1-C4-alkyl, -alkenyl, preferably hydrogen, with Nu⁻ being an anion of Nu.

3. Use of the biomolecule as halohydrin dehalogenase in conversion of at least a part of the amount of halohydrin of formula (I) into an epoxide of formula (II) and/or in the conversion of at least a part of an epoxide of formula (II) into an alcohol of formula (III) according to claim 1 or 2, **characterized in that** each of R₁, R₂, R₃, R₄, which does not form a cyclic structure with another of R₁, R₂, R₃, R₄ may have at least one carbon atom replaced by a hetero atom.

4. Use of the biomolecule as halohydrin dehalogenase in conversion of at least a part of the amount of halohydrin of formula (I) into an epoxide of formula (II) according to claim 1 wherein the halohydrin of formula (I) is selected from 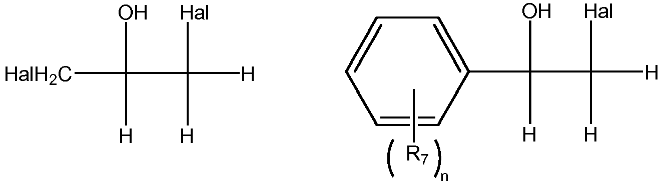 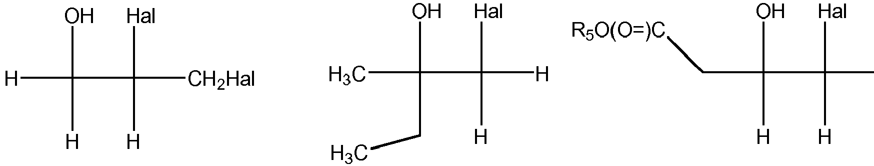 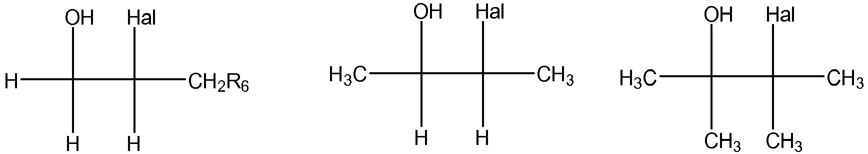 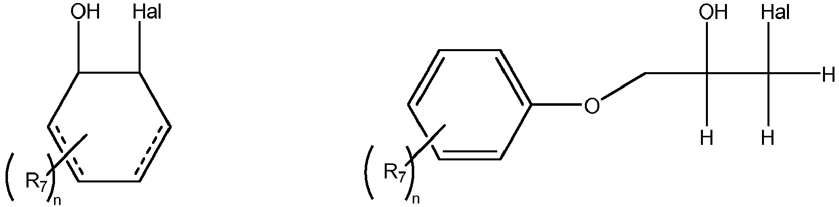 ,
wherein Hal is Cl or Br and is in case of more than one Hal preferably identical per substance, and R₇ is selected from halogen (F, Cl, Br, I) or C1-C3-alkyl and each ----- independently represents a double or single bond and n is zero or an integer between 1 and 4, preferably 0, 1 or 2.

5. Use of the biomolecule as halohydrin dehalogenase in conversion of at least a part of the epoxide of formula (II) into an alcohol of formula (III) according to claim 2 wherein the epoxide of formula (II) is selected from 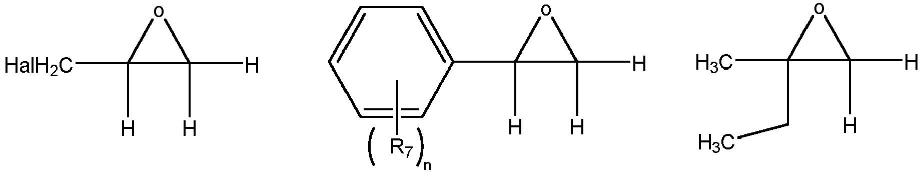 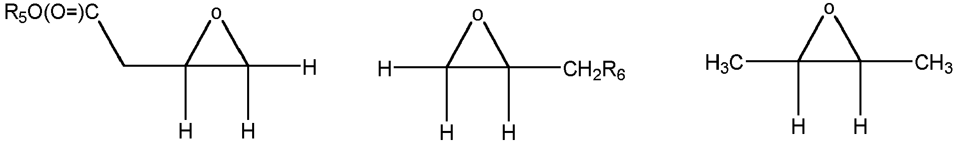 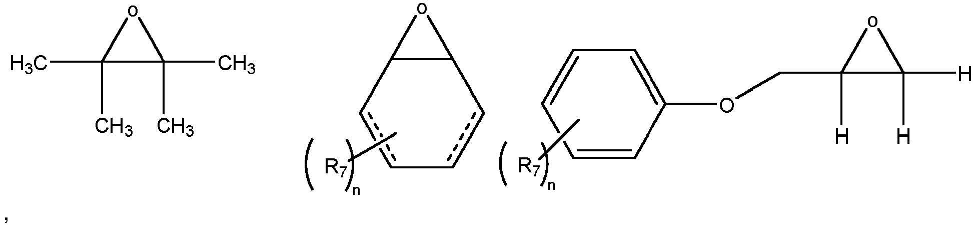 wherein Hal is Cl or Br and is in case of more than one Hal preferably identical per substance, and R₇ is selected from hydrogen, halogen (F, Cl, Br, I) or C1-C3-alkyl and each ----- independently represents a double or single bond and n is zero or an integer between 1 and 4, preferably 0, 1 or 2.

## Patentansprüche

1. Verwendung eines Biomoleküls mit der Proteinsequenz oder codierenden DNA-Sequenz von HheD3 mit der NCBI GenBank Accession Number ABM93639 als Halogenhydrindehalogenase bei der Umwandlung mindestens eines Teils der Menge eines Halogenhydrins der Formel (I) in ein Epoxid der Formel (II) 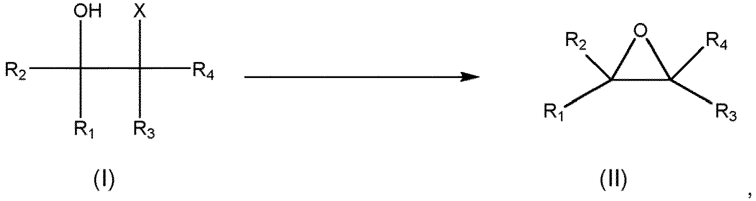 wobei
X für ein Halogenatom, das aus Cl, Br und I ausgewählt ist, steht;
R₁, R₂, R₃ und R₄ unabhängig für Wasserstoff, Halogen (F, Cl, Br, I) oder substituiertes oder unsubstituiertes C1-C12-Alkyl, C2-C12-Alkenyl, C2-C12-Alkinyl, C1-C12-Alkoxy, C3-C12-Cycloalkyl, C3-C12-Cycloalkenyl, C6-C12-Cycloalkinyl oder C5-C12-Aryl oder C1-C3-Alkyl-C(=O)-O-R₅ oder -C1-C3-Alkyl-O-R₅ stehen, wobei
R₅ aus Wasserstoff, C1-C6-Alkyl, C2-C6-Alkenyl, C2-C6-Alkinyl, C1-C6-Alkoxy, C5-C13-Alkylaryl, C5-C13-Arylalkyl, C5-C13-Aryloxy, C5-C13-Arylalkoxy, C5-C13-Heteroalkyl, C5-C13-Aryl, C5-C13-Heteroaryl, C3-C12-Cycloalkyl, C3-C12-Cycloalkenyl, C6-C12-Cycloalkinyl oder cyclischem C5-C13-Heteroalkyl ausgewählt ist, wobei im Fall von mehr als einem aliphatischen oder aromatischen Ringsystem die Ringsysteme kondensiert oder getrennt sind und R₅ mindestens einen weiteren Substituenten R₆ aufweisen kann, wobei
R₆ aus Wasserstoff, Hydroxyl, -C(=O)NH₂, Halogen (F, Cl, Br, I), -C1-C3-Alkyl, -C(=)O-C1-C3-Alkyl,-C(=)O-C1-C3-Alkyl, -NH-C(=)O-C1-C3-Alkyl, -C1-C3-Alkyl-O-C1-C3-alkyl, C2-C3-Alkenyl, -O-C2-C3-Alkenyl, C5-C7-Cycloalkyl, cyclischem C5-C7-Heteroalkyl, C5-C7-Aryl oder C5-C7-Heteroaryl ausgewählt ist,
und/oder
zwei von R₁, R₂, R₃ und R₄ zusammen eine cyclische Struktur bilden, die aus C3-C12-Cycloalkyl, C4-C12-Cycloalkenyl, C6-C12-Cycloalkinyl, cyclischem C3-C12-Heteroalkyl, cyclischem C4-C12-Heteroalkenyl oder cyclischem C6-C12-Heteroalkinyl, vorzugsweise einer C4-C8-Cycloalkyl- oder C4-C8-Cycloalkenylgruppe, weiter bevorzugt einer C5-C7-Cycloalkyl- oder C5-C7-Cycloalkenylgruppe, ganz besonders bevorzugt einer C5-C6-Cycloalkyl- oder C5-C6-Cycloalkenylgruppe ausgewählt ist, ausgewählt ist, wobei im Fall von mehr als einem Ringsystem die Ringsysteme kondensiert oder getrennt sind und wobei die Zahl der C-Atome, wie sie für die cyclische Struktur angegeben ist, immer die 2-C-Atome der Halogenhydrinstruktur, die das OH und das X tragen, einschließt, und
wobei jede cyclische Gruppe in R₁, R₂, R₃ und R₄ oder durch zwei von R₁, R₂, R₃ und R₄ gebildete cyclische Struktur gegebenenfalls mindestens einen weiteren Substituenten, der von Wasserstoff verschieden ist, aufweist, der vorzugsweise aus R₆, ganz besonders bevorzugt Halogen (F, Cl, Br, I) oder C1-C3-Alkyl (R₇), ausgewählt ist.

2. Verwendung eines Biomoleküls mit der Proteinsequenz oder codierenden DNA-Sequenz von HheD3 mit der NCBI GenBank Accession Number ABM93639 als Halogenhydrindehalogenase bei der Umwandlung mindestens eines Teils eines Epoxids der Formel (II) in einen Alkohol der Formel (III) 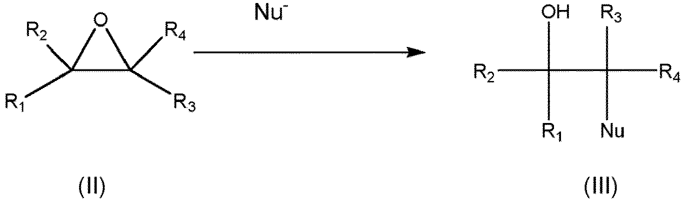 wobei
R₁, R₂, R₃ und R₄ unabhängig für Wasserstoff, Halogen (F, Cl, Br, I) oder substituiertes oder unsubstituiertes C1-C12-Alkyl, C2-C12-Alkenyl, C2-C12-Alkinyl, C1-C12-Alkoxy, C3-C12-Cycloalkyl, C3-C12-Cycloalkenyl, C6-C12-Cycloalkinyl oder C5-C12-Aryl oder C1-C3-Alkyl-C(=O)-O-R₅ oder -C1-C3-Alkyl-O-R₅ stehen, wobei
R₅ aus Wasserstoff, C1-C6-Alkyl, C2-C6-Alkenyl, C2-C6-Alkinyl, C1-C6-Alkoxy, C5-C13-Alkylaryl, C5-C13-Arylalkyl, C5-C13-Aryloxy, C5-C13-Arylalkoxy, C5-C13-Heteroalkyl, C5-C13-Aryl, C5-C13-Heteroaryl, C3-C12-Cycloalkyl, C3-C12-Cycloalkenyl, C6-C12-Cycloalkinyl oder cyclischem C5-C13-Heteroalkyl ausgewählt ist, wobei im Fall von mehr als einem aliphatischen oder aromatischen Ringsystem die Ringsysteme kondensiert oder getrennt sind und R₅ mindestens einen weiteren Substituenten R₆ aufweisen kann, wobei
R₆ aus Wasserstoff, Hydroxyl, -C(=O)NH₂, Halogen (F, Cl, Br, I), -C1-C3-Alkyl, -C(=)O-C1-C3-Alkyl,-C(=)O-C1-C3-Alkyl, -NH-C(=)O-C1-C3-Alkyl, -C1-C3-Alkyl-O-C1-C3-alkyl, C2-C3-Alkenyl, -O-C2-C3-Alkenyl, C5-C7-Cycloalkyl, cyclischem C5-C7-Heteroalkyl, C5-C7-Aryl oder C5-C7-Heteroaryl ausgewählt ist,
und/oder
zwei von R₁, R₂, R₃ und R₄ zusammen eine cyclische Struktur bilden, die aus C3-C12-Cycloalkyl, C4-C12-Cycloalkenyl, C6-C12-Cycloalkinyl, cyclischem C3-C12-Heteroalkyl, cyclischem C4-C12-Heteroalkenyl oder cyclischem C6-C12-Heteroalkinyl, vorzugsweise einer C4-C8-Cycloalkyl- oder C4-C8-Cycloalkenylgruppe, weiter bevorzugt einer C5-C7-Cycloalkyl- oder C5-C7-Cycloalkenylgruppe, ganz besonders bevorzugt einer C5-C6-Cycloalkyl- oder C5-C6-Cycloalkenylgruppe ausgewählt ist, ausgewählt ist, wobei im Fall von mehr als einem Ringsystem die Ringsysteme kondensiert oder getrennt sind und wobei die Zahl der C-Atome, wie sie für die cyclische Struktur angegeben ist, immer die 2-C-Atome der Halogenhydrinstruktur, die das OH und das X tragen, einschließt, und
wobei jede cyclische Gruppe in R₁, R₂, R₃ und R₄ oder durch zwei von R₁, R₂, R₃ und R₄ gebildete cyclische Struktur gegebenenfalls mindestens einen weiteren Substituenten, der von Wasserstoff verschieden ist, aufweist, der vorzugsweise aus R₆, ganz besonders bevorzugt Halogen (F, Cl, Br, I) oder C1-C3-Alkyl (R₇), ausgewählt ist,
und
Nu aus R'NO₂, N₃, NO₂, SCN, OCN, CN, Halogen (F, Cl, Br, I) und R"CO₂ ausgewählt ist, wobei
R' aus C1-C4-Alkyl, -Alkenyl, vorzugsweise CH₃, ausgewählt ist,
R" aus Wasserstoff, C1-C4-Alkyl, -Alkenyl, vorzugsweise Wasserstoff, ausgewählt ist,
wobei Nu⁻ ein Anion von Nu ist.

3. Verwendung des Biomoleküls als Halogenhydrindehalogenase bei der Umwandlung mindestens eines Teils der Menge von Halogenhydrin der Formel (I) in ein Epoxid der Formel (II) und/oder bei der Umwandlung mindestens eines Teils eines Epoxids der Formel (II) in einen Alkohol der Formel (III) nach Anspruch 1 oder 2, **dadurch gekennzeichnet, dass** bei jedem der Reste R₁, R₂, R₃ und R₄, der nicht mit einem anderen der Reste R₁, R₂, R₃ und R₄ eine cyclische Struktur bildet, mindestens ein Kohlenstoffatom durch ein Heteroatom ersetzt sein kann.

4. Verwendung des Biomoleküls als Halogenhydrindehalogenase bei der Umwandlung mindestens eines Teils der Menge von Halogenhydrin der Formel (I) in ein Epoxid der Formel (II) nach Anspruch 1, wobei das Halogenhydrin der Formel (I) aus 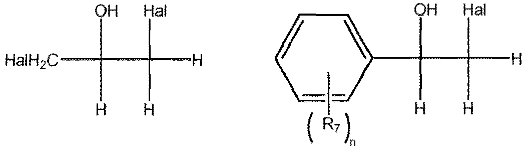 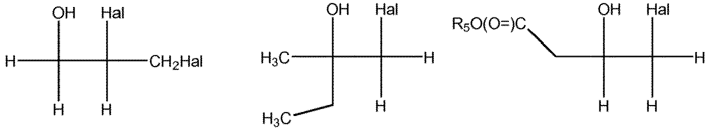 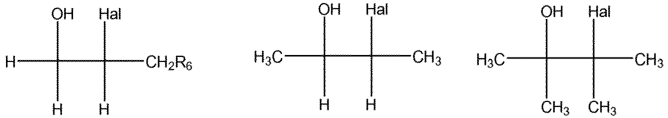 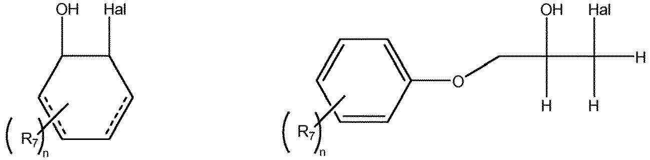 ausgewählt wird, wobei Hal für Cl oder Br steht und im Fall von mehr als einem Hal vorzugsweise pro Substanz gleich ist und R₇ aus Halogen (F, Cl, Br, I) oder C1-C3-Alkyl ausgewählt ist und ----- jeweils unabhängig für eine Doppel- oder Einfachbindung steht und n für null oder eine ganze Zahl zwischen 1 und 4, vorzugsweise 0, 1 oder 2, steht.

5. Verwendung des Biomoleküls als Halogenhydrindehalogenase bei der Umwandlung mindestens eines Teils des Epoxids der Formel (II) in einen Alkohol der Formel (III) nach Anspruch 2, wobei das Epoxid der Formel (II) aus 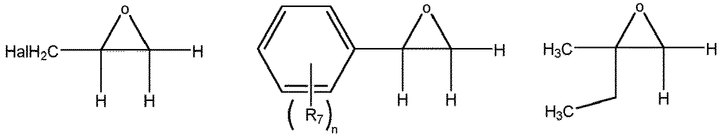 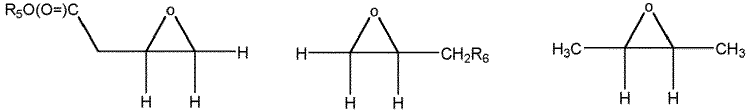 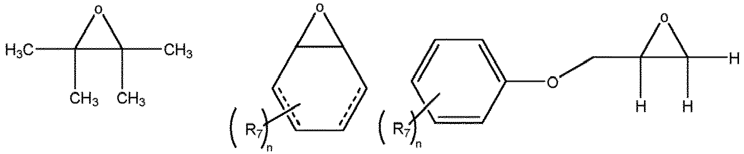 ausgewählt wird, wobei Hal für Cl oder Br steht und im Fall von mehr als einem Hal vorzugsweise pro Substanz gleich ist und R₇ aus Wasserstoff, Halogen (F, Cl, Br, I) oder C1-C3-Alkyl ausgewählt ist und ----- jeweils unabhängig für eine Doppel- oder Einfachbindung steht und n für null oder eine ganze Zahl zwischen 1 und 4, vorzugsweise 0, 1 oder 2, steht.

## Revendications

1. Utilisation d'une biomolécule ayant la séquence protéique de HheD3, ou la séquence d'ADN codante, ayant le numéro d'ordre GenBank ABM93639 du NCBI, comme halohydrine déshalogénase dans la conversion d'au moins une partie de la quantité d'une halohydrine de formule (I) en un époxyde de formule (II) 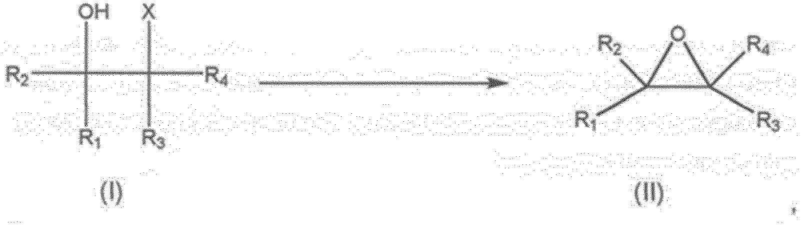 dans laquelle
X est un atome d'halogène choisi parmi Cl, Br, I ;
R₁, R₂, R₃, R₄ sont indépendamment hydrogène, halogène (F, Cl, Br, I) ou bien C1-C12-alkyle, C2-C12-alcényle, C2-C12-alcynyle, C1-C12-alcoxy, C3-C12-cycloalkyle, C3-C12-cycloalcényle, C6-C12-cycloalcynyle ou C5-C 12-aryle, substitué ou non substitué,
ou bien C1-C3-alkyle-C(=O)-O-R₅ ou -C1-C3-alkyl-O-R₅, avec
R₅ étant choisi parmi hydrogène, C1-C6-alkyle, C2-C6-alcényle, C2-C6-alcynyle, C1-C6-alcoxy, C5-C13-alkylaryle, C5-C13-arylalkyle, C5-C13-aryloxy, C5-C13-arylalcoxy, C5-C13-hétéroalkyle, C5-C13-aryle, C5-C13-hétéroaryle, C3-C12-cycloalkyle, C3-C12-cycloalcényle, C6-C12-cycloalcynyle ou C5-C13-hétéroalkyle cyclique, dans laquelle, dans le cas où il y aurait plus d'un système de noyau aliphatique ou aromatique, les systèmes de noyaux sont condensés ou séparés et R₅ peut avoir au moins un substituent supplémentaire R₆, avec
R₆ étant choisi parmi hydrogène, hydroxyle,-C(=O)-NH₂, halogène (F, Cl, Br, I), -C1-C3-alkyle, -C(=)O-C1 -C3-alkyle, -C(=)O-C1-C3-alkyle, -NH-C(=)0-Cl-C3-alkyle, -C1-C3-alkyl-O-C1-C3-alkyle, C2-C3-alcényle, -O-C2-C3-alcényle, C5-C7-cycloalkyle, C5-C7-hétéroalkyle cyclique, C5-C7-aryle ou C5-C7-hétéroaryle
et/ou
deux des R₁, R₂, R₃, R₄ forment ensemble une structure cyclique choisie parmi un groupe C3-C12-cycloalkyle, C4-C12-cycloalcényle, C6-C12-cycloalcynyle, C3-C12-hétéroalkyle cyclique, C4-C12-hétéroalcényle cyclique ou C6-C12-hétéroalcynyle cyclique, de préférence un groupe C4-C8-cycloalkyle ou C4-C8-cycloalcényle, mieux préféré un groupe C5-C7-cycloalkyle ou C5-C7-cycloalcényle, de manière préférée entre toutes un groupe C5-C6-cycloalkyle ou C5-C6-cycloalcényle, dans laquelle, dans le cas où il y aurait plus d'un système de noyau, les systèmes de noyaux sont condensés ou séparés, et dans laquelle le nombre d'atomes de C, tel qu'indiqué pour la structure cyclique, inclut toujours les 2 atomes de C de la structure d'halohydrine renfermant l'OH et le X, et dans laquelle chaque groupe cyclique dans les R₁, R₂, R₃, R₄ ou bien la structure cyclique formée par deux des R₁, R₂, R₃, R₄ a, en option, au moins un substituent supplémentaire différent de l'hydrogène, de préférence choisi à partir de R₆, de manière préférée entre toutes un halogène (F, Cl, Br, I) ou un -C1-C3-alkyle (R₇).

2. Utilisation d'une biomolécule ayant la séquence protéique de HheD3, ou la séquence d'ADN codante, ayant le numéro d'ordre GenBank ABM93639 du NCBI, comme halohydrine déshalogénase dans la conversion d'au moins une partie d'un époxyde de formule (II) en un alcool de formule (III) 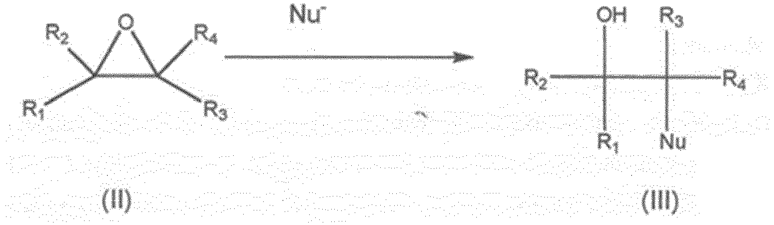 dans laquelle
R₁, R₂, R₃, R₄ sont indépendamment hydrogène, halogène (F, Cl, Br, I) ou bien C1-C12-alkyle, C2-C12-alcényle, C2-C12-alcynyle, C1-C12-alcoxy, C3-C12-cycloalkyle, C3-C12-cycloalcényle, C6-C12-cycloalcynyle ou C5-C 12-aryle, substitué ou non substitué,
ou bien -C1-C3-alkyl-C(=O)-O-R₅ ou -C1-C3-alkyl-O-R₅, avec
R₅ étant choisi parmi hydrogène, C1-C6-alkyle, C2-C6-alcényle, C2-C6-alcynyle, C1-C6-alcoxy, C5-C13-alkylaryle, C5-C13-arylalkyle, C5-C13-aryloxy, C5-C13-arylalcoxy, C5-C13-hétéroalkyle, C5-C13-aryle, C5-C13-hétéroaryle, C3-C12-cycloalkyle, C3-C12-cycloalcényle, C6-C12-cycloalcynyle ou C5-C13-hétéroalkyle cyclique, dans laquelle, dans le cas où il y aurait plus d'un système de noyau aliphatique ou aromatique, les systèmes de noyaux sont condensés ou séparés et R₅ peut avoir au moins un substituent supplémentaire R₆, avec
R₆ étant choisi parmi hydrogène, hydroxyle,-C(=O)-NH₂, halogène (F, Cl, Br, I), -C1-C3-alkyle, -C(=)O-C1 -C3-alkyle, -C(=)O-C1-C3-alkyle, -NH-C(=)O-C1-C3-alkyle, -C1-C3-alkyl-O-C1-C3-alkyle, C2-C3-alcényle, -O-C2-C3-alcényle, C5-C7-cycloalkyle, C5-C7-hétéroalkyle cyclique, C5-C7-aryle ou C5-C7-hétéroaryle
et/ou
deux des R₁, R₂, R₃, R₄ forment ensemble une structure cyclique choisie parmi un groupe C3-C12-cycloalkyle, C4-C12-cycloalcényle, C6-C12-cycloalcynyle, C3-C12-hétéroalkyle cyclique, C4-C12-hétéroalcényle cyclique ou C6-C12-hétéroalcynyle cyclique, de préférence un groupe C4-C8-cycloalkyle ou C4-C8-cycloalcényle, mieux préféré un groupe C5-C7-cycloalkyle ou C5-C7-cycloalcényle, de manière préférée entre toutes un groupe C5-C6-cycloalkyle ou C5-C6-cycloalcényle, dans laquelle, dans le cas où il y aurait plus d'un système de noyau, les systèmes de noyaux sont condensés ou séparés, et dans laquelle le nombre d'atomes de C, tel qu'indiqué pour la structure cyclique, inclut toujours les 2 atomes de C de la structure d'halohydrine renfermant l'OH et le X, et dans laquelle chaque groupe cyclique dans les R₁, R₂, R₃, R₄ ou bien la structure cyclique formée par deux des R₁, R₂, R₃, R₄ a, en option, au moins un substituent supplémentaire différent de l'hydrogène, de préférence choisi à partir de R₆, de manière préférée entre toutes un halogène (F, Cl, Br, I) ou un C1-C3-alkyle (R₇)
et
Nu est choisi parmi les R'NO₂, N₃, NO₂, SCN, OCN, CN, halogène (F, Cl, Br, I) et R"CO₂, dans laquelle
R' est choisi parmi C1-C4-alkyle, -alcényle, de préférence CH₃,
R" est choisi parmi hydrogène, C1-C4-alkyle, -alcényle, de préférence l'hydrogène,
avec Nu⁻ étant un anion de Nu.

3. Utilisation de la biomolécule comme halohydrine déshalogénase dans la conversion d'au moins une partie de la quantité d'une halohydrine de formule (I) en un époxyde de formule (II) et/ou dans la conversion d'au moins une partie d'un époxyde de formule (II) en un alcool de formule (III) selon les revendications 1 ou 2, **caractérisée en ce que** chacun des R₁, R₂, R₃, R₄, qui ne forme pas une structure cyclique avec un autre des R₁, R₂, R₃, R₄, peut avoir au moins un atome de carbone remplacé par un hétéroatome.

4. Utilisation de la biomolécule comme halohydrine déshalogénase dans la conversion d'au moins une partie de la quantité d'une halohydrine de formule (I) en un époxyde de formule (II) selon la revendication 1, dans laquelle l'halohydrine de formule (I) est choisie parmi 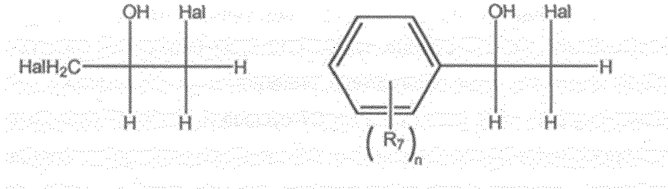 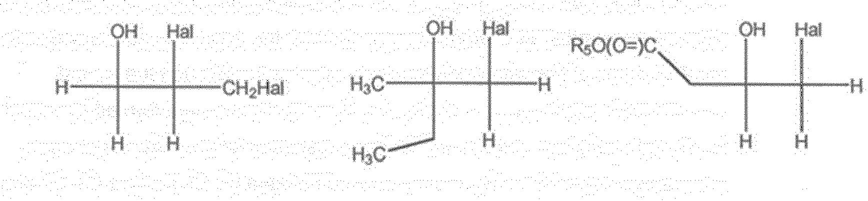 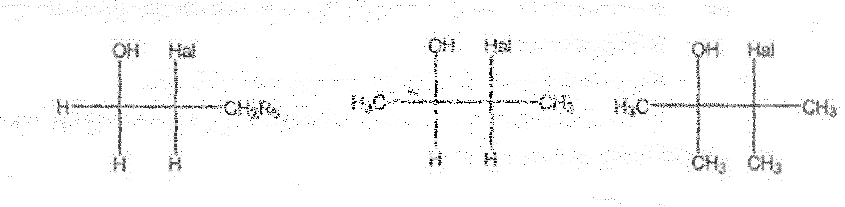 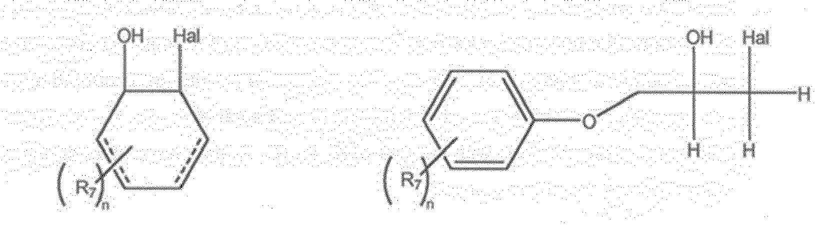 dans laquelle Hal est Cl ou Br et, dans le cas où il y aurait plus d'un Hal, il est de préférence identique en substance, et R₇ est choisi parmi halogène (F, Cl, Br, I) ou C1-C3-alkyle et chaque 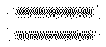 représente indépendamment une double ou une simple liaison et n est zéro ou un entier compris entre 1 et 4, de préférence 0, 1 ou 2.

5. Utilisation de la biomolécule comme halohydrine déshalogénase dans la conversion d'au moins une partie del'époxyde de formule (II) en un alcool de formule (III) selon la revendication 2, dans laquelle l'époxyde de formule (III) est choisi parmi 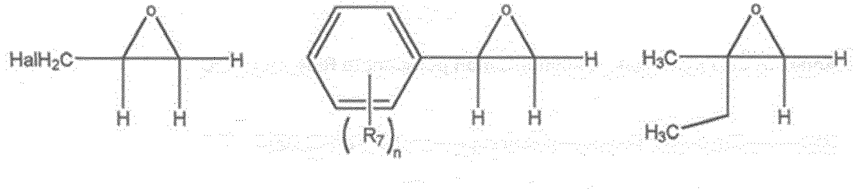 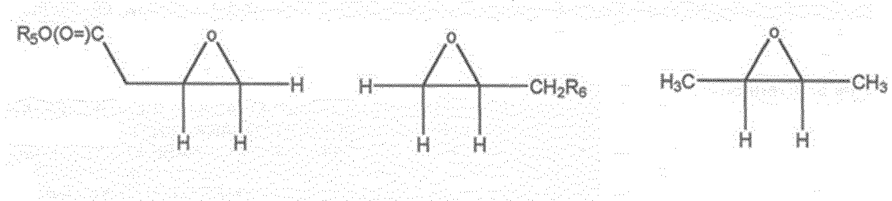 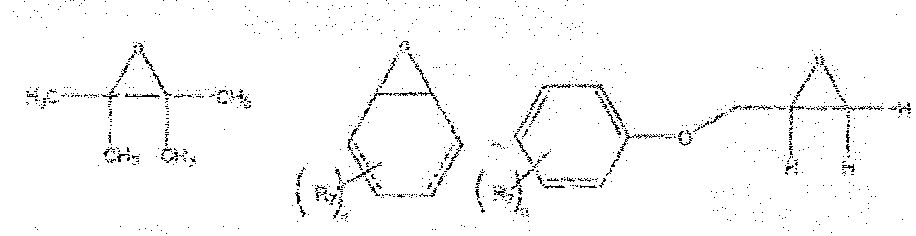 dans laquelle Hal est Cl ou Br et, dans le cas où il y aurait plus d'un Hal, il est de préférence identique en substance, et R₇ est choisi parmi hydrogène, halogène (F, Cl, Br, I) ou C1-C3-alkyle et chaque 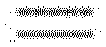 représente indépendamment une double ou une simple liaison et n est zéro ou un entier compris entre 1 et 4, de préférence 0, 1 ou 2.
